(19) Europäisches Patentamt
European Patent Office
Office européen des brevets

(11) EP 3 230 261 B1

(12) # EUROPÄISCHE PATENTSCHRIFT

(45) Veröffentlichungstag und Bekanntmachung des Hinweises auf die Patenterteilung:
**05.09.2018 Patentblatt 2018/36**

(21) Anmeldenummer: **15807900.4**

(22) Anmeldetag: **09.12.2015**

(51) Int Cl.:
***C07C 271/30*** (2006.01) ***C07C 271/48*** (2006.01)
***G03F 7/00*** (2006.01) ***G03F 7/027*** (2006.01)
***G03F 7/035*** (2006.01)

(86) Internationale Anmeldenummer:
**PCT/EP2015/079152**

(87) Internationale Veröffentlichungsnummer:
**WO 2016/091965 (16.06.2016 Gazette 2016/24)**

(54) **NAPHTHYLACRYLATE ALS SCHREIBMONOMERE FÜR PHOTOPOLYMERE**

NAPHTHYL ACRYLATE AS WRITE MONOMERS FOR PHOTOPOLYMERS

ACRYLATE NAPHTYLE COMME MONOMÈRE D'ÉCRITURE POUR PHOTO-POLYMÈRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2014 EP 14197565**
**22.06.2015 EP 15173157**

(43) Veröffentlichungstag der Anmeldung:
**18.10.2017 Patentblatt 2017/42**

(73) Patentinhaber: **Covestro Deutschland AG**
**51373 Leverkusen (DE)**

(72) Erfinder:
- **RÖLLE, Thomas**
  **51381 Leverkusen (DE)**
- **BERNETH, Horst**
  **51373 Leverkusen (DE)**
- **BRUDER, Friedrich-Karl**
  **47802 Krefeld (DE)**
- **HÖNEL, Dennis**
  **53909 Zülpich-Wichterich (DE)**
- **KOSTROMINE, Serguei**
  **53913 Swisttal-Buschhofen (DE)**
- **FÄCKE, Thomas**
  **51375 Leverkusen (DE)**

(74) Vertreter: **Levpat**
**c/o Covestro AG**
**Gebäude 4825**
**51365 Leverkusen (DE)**

(56) Entgegenhaltungen:
**WO-A2-2011/054818**

- **LEE Y-K ET AL: "Synthesis of novel chiral poly(methacrylate)s bearing urethane and cinchona alkaloid moieties in side chain and their chiral recognition abilities", POLYMER, ELSEVIER SCIENCE PUBLISHERS B.V, GB, Bd. 43, Nr. 26, 1. Dezember 2002 (2002-12-01), Seiten 7539-7547, XP004393272, ISSN: 0032-3861, DOI: 10.1016/S0032-3861(02)00710-3**
- **DATABASE WPI Week 201311 Thomson Scientific, London, GB; AN 2013-B24859 XP002739534, & JP 2013 014533 A (KAWASAKI KASEI KOGYO KK) 24. Januar 2013 (2013-01-24)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 230 261 B1

## Beschreibung

Die Erfindung betrifft Naphthylacrylate, die insbesondere als Schreibmonomere in Photopolymer-Formulierungen für holographische Medien verwendet werden können. Darüber hinaus sind eine Photopolymer-Formulierung, umfassend Matrixpolymere, Schreibmonomere und Photoinitiatoren, wobei die Schreibmonomere ein erfindungsgemäßes Naphthylacrylat umfassen, ein holographisches Medium, umfassend Matrixpolymere, Schreibmonomere und Photoinitiatoren, wobei die Schreibmonomere ein erfindungsgemäßes Naphthylacrylat umfassen, sowie ein Display umfassend ein erfindungsgemäßes holographisches Medium Gegenstände der Erfindung.

Photopolymer-Formulierungen sind im Stand der Technik bekannt. So ist beispielsweise in der EP 2 154 128 eine Photopolymer-Formulierung beschrieben, die Polyurethan-basierte Matrixpolymere, Schreibmonomere auf Acrylatbasis sowie Photoinitiatoren enthält. Im ausgehärtetem Zustand sind in der Polyurethanmatrix die Schreibmonomere und die Photoinitiatoren räumlich isotrop verteilt eingebettet. Dokument P2013014533 beschreibt ähnliche Verbindungen zur Verwendung bei der Polymerisation. wo2011054818 beschreibt ähnliche Verbindungen zur Verwendung bei der Photopolymerisation. Für die Verwendungen von Photopolymer-Formulierungen spielt die durch die holographische Belichtung im Photopolymer erzeugte Brechungsindexmodulation $\Delta n$ die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylate an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im Folgenden DE wie Diffraction Efficiency, genannt. Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die Lichtmenge des Referenzlichtes die notwendig ist, um das Signal mit einer festen Helligkeit sichtbar zu machen.

Hochbrechende Acrylate sind in der Lage, Brechungsindexgitter mit hoher Amplitude zwischen Bereichen mit niedrigem Brechungsindex und Bereichen mit hohem Brechungsindex zu erzeugen und damit in Photopolymeren Hologramme mit hohem DE und hohem $\Delta n$ zu ermöglichen. Dabei ist zu beachten, dass die DE vom Produkt aus $\Delta n$ und der Photopolymerschichtdicke d abhängt. Je größer das Produkt, desto größer die mögliche DE (für Reflexionshologramme). Die Breite des Winkelbereiches bei dem das Hologramm z.B. bei monochromatischer Beleuchtung sichtbar (rekonstruiert) wird, hängt nur von der Schichtdicke d ab.

Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt je kleiner d desto größer die jeweiligen Akzeptanzbreiten. Will man daher helle und leicht sichtbare Hologramme herstellen, ist ein hohes $\Delta n$ und eine geringe Dicke d anzustreben und zwar so, dass die DE möglichst groß wird. Das heißt je höher $\Delta n$ wird, desto mehr Freiraum zur Gestaltung der Schichtdicke d für helle Hologramme erreicht man ohne Verlust an DE. Daher kommt der Optimierung von $\Delta n$ bei der Optimierung von Photopolymer-Formulierungen eine herausragenden Bedeutung zu (P. Hariharan, Optical Holography, 2nd Edition, Cambridge University Press, 1996).

Aufgabe der vorliegenden Erfindung war es, eine Verbindung bereitzustellen, die sich als Schreibmonomer zur Herstellung von holographischen Medien mit hohem Brechungsindexkontrast ($\Delta n$) eignet. Diese Aufgabe ist durch eine Verbindung der Formel (I)

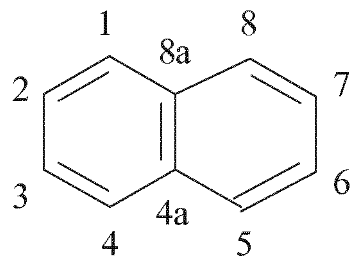

Formel (I)

a) die an wenigstens einem der Kohlenstoffatome 1, 2, 3, 4, 5, 6, 7, 8 mit einem Rest $R_{Arcyl}$ der Formel (II) substituiert ist,

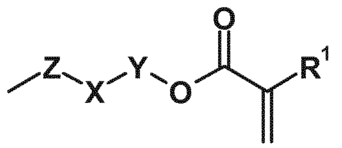

Formel (II)

wobei in der Formel (II)

$R^1$ Wasserstoff oder eine $(C_1-C_6)$-Alkylgruppe,

X eine Carbonamid (-C(O)N-) oder eine Carbonester (-C(O)O-) oder eine Sulfonamid ($-SO_2N-$) Gruppe,

Y ein gesättigter oder ungesättigter oder linearer oder verzweigter gegebenenfalls substituierter 2-10 C-Atome aufweisender Rest oder ein bis zu fünf ($-CH_2-CH_2-O-$)- oder ($-C(CH_3)H-CH_2-O-$)-Gruppen aufweisender Polyether oder ein bis zu fünf Stickstoff-Atome aufweisendes Polyamin, und

Z Sauerstoff oder Schwefel ist,

b) und die Verbindung der Formel (I) an wenigstens einem weiteren Kohlenstoffatom 1, 2, 3, 4, 5, 6, 7, 8 mit einem Rest der Formel (III) substituiert ist

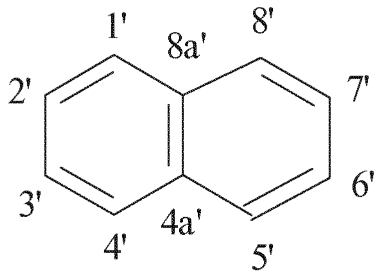

Formel (III)

wobei in der Formel (III)
die Kohlenstoffatome der Verbindung der Formel (III) jeweils unabhängig mit Wasserstoff, Halogen, einer Cyano-Gruppe, einer Nitro-Gruppe oder einer gegebenenfalls substituierten Alkyl-, Alkenyl-, Alkinyl-, Aralkyl-, Aryl- oder Heteroaryl-Gruppe oder einer gegebenenfalls substituierten Alkylthio-Gruppe oder einer beliebig substituierten Carbamoyl-Gruppe, die auch verbrückend an einen Rest der Formel (I) gebunden sein kann, oder einer Trifluormethyl-Gruppe oder einer Trifluormethoxy-Gruppe oder einem Rest $R_{Arcyl'}$ der Formel (IV),

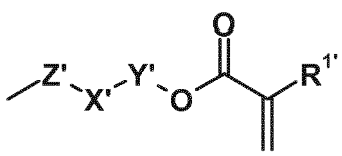

Formel (IV)

wobei in der Formel (IV)

$R^{1'}$ Wasserstoff oder eine (C1-C6)-Alkylgruppe,

X' eine Carbonamid (-C(O)N-) oder eine Carbonester (-C(O)O-) oder eine Sulfonamid ($-SO_2N-$) Gruppe,

Y' ein gesättigter oder ungesättigter oder linearer oder verzweigter gegebenenfalls substituierter 2-10 C-Atome aufweisender Rest oder ein bis zu fünf ($-CH_2-CH_2-O-$)- oder ($-C(CH_3)H-CH_2-O-$)-Gruppen aufweisender Polyether oder ein bis zu fünf Stickstoff-Atome aufweisendes Polyamin, und

Z' Sauerstoff oder Schwefel ist,

substituiert sind und

c) die übrigen Kohlenstoffatome der Verbindung der Formel (I) jeweils unabhängig mit Wasserstoff, Halogen, einer Cyano-Gruppe, einer Nitro-Gruppe oder einer gegebenenfalls substituierten Alkyl-, Alkenyl-, Alkinyl-, Aralkyl-, Aryl- oder Heteroaryl-Gruppe oder einer gegebenenfalls substituierten Alkylthio-Gruppe oder einer Trifluormethyl-Gruppe oder einer Trifluormethoxy-Gruppe substituiert sind, gelöst.

So wurde überraschenderweise gefunden, dass sich das Verbindungen der Formel (I) sehr gut als Schreibmonomer zur Herstellung von holographischen Medien mit sehr hohem Brechungsindexkontrast ($\Delta n$) und hoher optischer Qualität eignet.

Die erfindungsgemäßen Verbindungen der Formel (I) können beispielsweise in der folgenden Weise hergestellt werden: Durch oxidative Kupplung von Naphtholen können bezüglich Formel (I) und Formel (III) symmetrische oder unsymmetrische Bis-Naphthole hergestellt und gegebenenfalls präparativ getrennt werden, die dann in einem nachfolgenden Syntheseschritt mit OH-reaktiven Verbindungen zu Acrylaten umgesetzt werden können. Es ist ebenfalls möglich, kommerziell erhältliche Bis-Naphthole mit OH-reaktiven Verbindungen zu Acrylaten umzusetzen. Darüber hinaus können auch verbrückende zwei- oder höherfunktionelle OH-reaktive Bausteine zur Kupplung von zwei Bis-Naphtholen eingesetzt werden und die erhaltenen Produkte dann weiter zu Acrylaten der Formel (I) umgesetzt werden.

Gemäß einer ersten bevorzugten Ausführungsform der erfindungsgemäßen Verbindung ist vorgesehen, dass sie an dem Kohlenstoffatom der Position 5 in der Formel (I) mit dem Rest der Formel (III) substituiert ist, wobei der Rest der Formel (III) bevorzugt über das Kohlenstoffatom der Position 8' an das Kohlenstoffatom der Position 5 gebunden sein kann.

Ebenfalls bevorzugt ist, wenn die Verbindung an dem Kohlenstoffatom der Position 6 in der Formel (I) mit dem Rest $R_{Arcyl}$ der Formel (II) substituiert ist.

Vorteilhaft ist auch, wenn der Rest der Formel (III) an dem Kohlenstoffatom der Position 7' mit dem Rest $R_{Arcyl'}$ der Formel (IV) substituiert ist.

Bei einer weiteren bevorzugten Ausführungsform der Erfindung ist vorgesehen, dass in dem Rest $R_{Arcyl}$ X für Carbonamid und / oder in dem Rest $R_{Arcyl'}$ X für Carbonamid steht.

Weiterhin vorteilhaft ist auch, wenn in dem Rest $R_{Arcyl}$ $R^1$ für Wasserstoff oder einen $CH_3$-Rest und / oder in dem Rest $R_{Arcyl'}$ $R^{1'}$ für Wasserstoff oder einen $CH_3$-Rest steht.

Darüber hinaus kann vorzugsweise in dem Rest $R_{Arcyl}$ Y ein -CH2-CH2-Rest und / oder in dem der Rest $R_{Arcyl'}$ Y' ein $-CH_2-CH_2$-Rest sein.

Bevorzugt ist auch, wenn Z und / oder Z' Sauerstoff ist.

Ganz besonders bevorzugt ist, wenn Z und / oder Z' Sauerstoff ist und X und / oder X' eine Carbonamid-Gruppe ist.

Insbesondere bevorzugt ist die erfindungsgemäße Verbindung der Formel (I) aus der Gruppe der folgenden Substanzen ausgewählt: 2-[({[2'-({[2-(Acryloyloxy)ethyl]carbamoyl}oxy)-1,1'-binaphthyl-2-yl]oxy}carbonyl)amino]ethyl methacrylat, Dimethyl 2,2'-bis({[2-(methacryloyloxy)ethyl]carbamoyl}oxy)-1,1'-binaphthyl-3,3'-dicarboxylat, Diethyl 2,2'-bis({[2-(methacryloyloxy)ethyl]carbamoyl}oxy)-1,1'-binaphthyl-3,3 '-dicarboxylat, 1,1'-Binaphthyl-2,2'-diylbis(oxycarbonyliminoethan-2,1-diyl)bisacrylat, 1,1'-Binaphthyl-2,2'-diylbis(oxycarbonyliminoethan-2,1-diyl)bis(2-methylacrylat), (6,6'-Dicyano-1,1'-binaphthyl-2,2'-diyl)bis(oxycarbonyliminoethan-2,1-diyl) bisacrylat, (6,6'-Difluoro-1,1'-binaphthyl-2,2'-diyl)bis(oxycarbonyliminoethan-2,1-diyl) bisacrylat, (6,6'-Dichloro-1,1'-binaphthyl-2,2'-diyl)bis(oxycarbonyliminoethan-2,1-diyl) bisacrylat(6,6'-Dibromo-1,1'-binaphthyl-2,2'-diyl)bis(oxycarbonyliminoethan-2,1-diyl) bisacrylat, (6,6'-Diiodo-1,1'-binaphthyl-2,2'-diyl)bis(oxycarbonyliminoethan-2,1-diyl) bisacrylat, Difluoro-1,1'-binaphthyl-2,2'-diyl)bis(oxycarbonyliminoethan-2,1-diyl) bis(2-methylacrylat), (6,6'-Dichloro-1,1'-binaphthyl-2,2'-diyl)bis(oxycarbonyliminoethan-2,1-diyl) bis(2-methylacrylat), (6,6'-Dibromo-1,1'-binaphthyl-2,2'-diyl)bis(oxycarbonyliminoethan-2,1-diyl) bis(2-methylacrylat), (6,6'-Diiodo-1,1'-binaphthyl-2,2'-diyl)bis(oxycarbonyliminoethan-2,1-diyl) bis(2-methylacrylat), (7,7'-Dimethoxy-1,1'-binaphthyl-2,2'-diyl)bis(oxycarbonyliminoethan-2,1-diyl) bisacrylat, (7,7'-Diethoxy-1,1'-binaphthyl-2,2'-diyl)bis(oxycarbonyliminoethan-2,1-diyl) bisacrylat, 2-{[({2'-[(Hexylcarbamoyl)oxy]-1,1'-binaphthyl-2-yl}oxy)carbonyl]amino}ethyl acrylat, 2-{[({2'-[(Butylcarbamoyl)oxy]-1,1'-binaphthyl-2-yl}oxy)carbonyl]amino}ethyl acrylat, 2-{[({2'-[(Hexylcarbamoyl)oxy]-1,1'-binaphthyl-2-yl}oxy)carbonyl]amino}ethyl 2-methylacrylat, 2-{[({2'-[(Butylcarbamoyl)oxy]-1,1'-binaphthyl-2-yl}oxy)carbonyl]amino}ethyl2-methylacrylat, 2-{[({2'-[(Hexylcarbamoyl)-oxy]-1,1'-binaphthyl-2-yl}oxy)carbonyl]amino}ethyl acrylat, 2-{[({2'-[(Hexylcarbamoyl)-oxy]-1,1'-binaphthyl-2-yl}oxy)carbonyl]amino}ethyl 2-methylacrylat, 2-{[({2'-[(Hexylcarbamoyl)oxy]-1,1'-binaphthyl-2-yl}oxy)carbonyl]amino}ethyl acrylat, 2-{[({2'-[(Hexylcarbamoyl)oxy]-1,1'-binaphthyl-2-yl)oxy)carbonyl]amino}ethyl 2-methylacrylat, 2-{[({2'-[(Butylcarbamoyl)oxy]-1,1'-binaphthyl-2-yl}oxy)carbonyl]amino}ethyl acrylat, 2-{[({2'-[(Butylcarbamoyl)oxy]-1,1'-binaphthyl-2-yl}oxy)carbonyl]amino}ethyl 2-methylacrylat, 2-[({[2'-({[3-(Methylsulfanyl)phenyl]carbamoyl}oxy)-1,1'-binaphthyl-2-yl]oxy}-carbonyl)amino]ethyl acrylat, 2-[({[2'-({[3-(Methylsulfanyl)phenyl]carbamoyl}oxy)-1,1'-binaphthyl-2-yl]oxy}carbonyl)amino]ethyl methacrylat, 2-[({[2'-({[2-(Methylsulfanyl)phenyl]carbamoyl}oxy)-1,1'-binaphthyl-2-yl]oxy}carbonyl)amino] ethyl acrylat, 2-[({[2'-({[2-(Methylsulfanyl)phenyl]carbamoyl}oxy)-1,1'-binaphthyl-2-yl]oxy}-carbonyl)amino]ethyl methacrylat,

2-[({[2'-({[4-(Methylsulfanyl)phenyl]carbamoyl}oxy)-1,1'-binaphthyl-2-yl]oxy}carbonyl)amino]ethyl acrylat, 2-[({[2'-({[4-(Methylsulfanyl)phenyl]carbamoyl}oxy)-1,1'-binaphthyl-2-yl]oxy}carbonyl)amino]ethyl methacrylat, 2-{[({2'-[(1-Naphthylcarbamoyl)oxy]-1,1'-binaphthyl-2-yl}oxy)carbonyl]amino}ethyl acrylat, 2-{[({2'-[(1-Naphthylcarbamoyl)oxy]-1,1'-binaphthyl-2-yl}oxy)carbonyl]amino}ethylmethacrylat, Hexan-1,6-diylbis(carbamoyloxy-1,1'-binaphthyle-2',2-diyloxycarbonyliminoethan-2,1-diyl) bisacrylat, Hexan-1,6-diylbis(carbamoyloxy-1,1'-binaphthyle-2',2-diyloxycarbonyliminoethan-2,1-diyl) bis(2-methylacrylat), (2,2,4-Trimethylhexane-1,6-diyl)bis(carbamoyloxy-1,1'-binaphthyl-2',2-diyloxycarbonyliminoethane-2,1-diyl)-bisacrylat, (2,2,4-Trimethylhexane-1,6-diyl)bis-(carbamoyloxy-1,1'-binaphthyl-2',2-diyloxycarbonyliminoethane-2,1-diyl)-bis(2-methylacrylat), 2-({[(2'-{[(3-{[({[2'-({[2-(Acryloyloxy)ethyl]carbamoyl}oxy)-1,1'-bi-naphthyl-2-yl]oxy}carbonyl)amino]methyl}-3,5,5-trimethylcyclohexyl)carbamoyl]-oxy}-1,1'-binaphthyl-2-yl)oxy]carbonyl}amino)ethyl acrylat, 2-({[(2'-{[(3-{[({[2'-({[2-(Methacryloyloxy)ethyl]carbamoyl}oxy)-1,1'-binaphthyl-2-yl]oxy}carbonyl)amino]methyl}-3,5,5-trimethylcyclohexyl)¬carbamoyl]oxy}-1,1'-binaphthyl-2-yl)oxy]carbonyl}amino)ethyl methacrylat), (6-Fluor-1,1'-binaphthyl-2,2'-diyl)bis(oxycarbonyliminoethan-2,1-diyl) bisacrylat, (6-Fluor-1,1'-binaphthyl-2,2'-diyl)bis(oxycarbonyliminoethan-2,1-diyl) bis (2-methylacrylat), (6-Chlor-1,1'-binaphthyl-2,2'-diyl)bis(oxycarbonyliminoethan-2,1-diyl) bisacrylat, (6-Chlor-1,1'-binaphthyl-2,2'-diyl)bis(oxycarbonyliminoethan-2,1-diyl) bis (2-methylacrylat), (6-Brom-1,1'-binaphthyl-2,2'-diyl)bis(oxycarbonyliminoethan-2,1-diyl) bisacrylat, (6-Brom-1,1'-binaphthyl-2,2'-diyl)bis(oxycarbonyliminoethan-2,1-diyl) bis (2-methylacrylat), (6-Iod-1,1'-binaphthyl-2,2'-diyl)bis(oxycarbonyliminoethan-2,1-diyl) bisacrylat, (6-Iod-1,1'-binaphthyl-2,2'-diyl)bis(oxycarbonyliminoethan-2,1-diyl) bis (2-methylacrylat), (6-Cyan-1,1'-binaphthyl-2,2'-diyl)bis(oxycarbonyliminoethan-2,1-diyl) bisacrylat, (6-Cyan-1,1'-binaphthyl-2,2'-diyl)bis(oxycarbonyliminoethan-2,1-diyl) bis(2-methylacrylat), (6-Fluor-6'-cyan-1,1'-binaphthyl-2,2'-diyl)bis(oxycarbonyliminoethan-2,1-diyl) bisacrylat, (6-Fluor-6'-cyan-1,1'-binaphthyl-2,2'-diyl)bis(oxycarbonyliminoethan-2,1-diyl) bis(2-methylacrylat), (6-Chlor-6'-cyan-1,1'-binaphthyl-2,2'-diyl)bis(oxycarbonyliminoethan-2,1-diyl)bisacrylat, (6-Chlor-6'-cyan-1,1'-binaphthyl-2,2'-diyl)bis(oxycarbonyliminoethan-2,1-diyl) bis(2-methylacrylat), (6-Brom-6'-cyan-1,1'-binaphthyl-2,2'-diyl)bis(oxycarbonyliminoethan-2,1-diyl) bisacrylat, (6-Brom-6'-cyan-1,1'-binaphthyl-2,2'-diyl)bis(oxycarbonyliminoethan-2,1-diyl) bis(2-methylacrylat), (6-Iod-6'-cyan-1,1'-binaphthyl-2,2'-diyl)bis(oxycarbonyliminoethan-2,1-diyl) bisacrylat, (6-Iod-6'-cyan-1,1'-binaphthyl-2,2'-diyl)bis(oxycarbonyliminoethan-2,1-diyl) bis(2-methylacrylat).

Ein weiterer Gegenstand der Erfindung ist eine Photopolymer-Formulierung umfassend Matrixpolymere, Schreibmonomere und Photoinitiatoren, wobei die Schreibmonomer eine erfindungsgemäße Verbindung gemäß Formel (I) umfassen.

Die Matrixpolymere der erfindungsgemäßen Photopolymer-Formulierung können insbesondere vernetzt und besonders bevorzugt dreidimensional vernetzt sein.

Vorteilhaft ist auch, wenn die Matrixpolymere Polyurethane sind, wobei die die Polyurethane insbesondere durch Umsetzung wenigstens einer Polyisocyanat-Komponente a) mit wenigstens einer Isocyanat-reaktiven-Komponente b) erhältlich sein können.

Die Polyisocyanat-Komponente a) umfasst bevorzugt wenigstens eine organische Verbindung mit wenigstens zwei NCO-Gruppen. Bei diesen organischen Verbindungen kann es sich insbesondere um monomere Di- und Triisocyanate, Polyisocyanate und / oder NCOfunktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diisocyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, $H_6$-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)-benzol (XDI) und / oder das analoge 1,4-Isomere oder beliebige Mischungen der vorgenannten Verbindungen.

Geeignete Polyisocyanate sind Verbindungen mit Urethan-, Harnstoff-, Carbodiimid-, Acylharnstoff-, Amid-, Isocyanurat-, Allophanat-, Biuret-, Oxadiazintrion-, Uretdion- und/oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder MercaptoVerbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und/oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal- Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, $\varepsilon$-Caprolacton und / oder Methyl-$\varepsilon$-caprolacton an hydroxyfunktionelle Verbindungen wie mehrwertige Alkohole einer OH-Funktionalität $\geq 2$ beispielsweise der nachstehend genannten Art erhalten werden können.

Beispiele für geeignete Alkohole sind alle mehrwertigen Alkohole wie z.B. die $C_2$- $C_{12}$-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität $\geq 2$, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität $\geq 2$ sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten $C_3$- und $C_4$-Isomere.

Daneben sind als Bestandteile der Polyol-Komponente b1) als polyfunktionelle, isocyanatreaktive Verbindungen auch niedermolekulare, d.h. mit Molekulargewichten $\leq 500$ g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungs-isomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipenta-erythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicylohexylmethandiamin, Isophorondiamin (IPDA), difunktionelle Polyamine wie z.B. die Jeffamine®, aminterminierte Polymere, insbesondere mit zahlenmittleren Molmassen $\leq 10000$ g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10000 g/Mol, weiter bevorzugt ≥ 500 und ≤ 8000 g/Mol und ganz besonders bevorzugt ≥ 800 und ≤ 5000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder Mercapto-Gruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Bei einer weiteren bevorzugten Ausführungsform ist vorgesehen, dass die Schreibmonomere ein mono- und / oder ein multifunktionelles (Meth)acrylat-Schreibmonomere umfassen. Ganz besonders bevorzugt können die Schreibmonomere zusätzlich wenigstens ein mono- und / oder ein multifunktionelles Urethan(meth)acrylat umfassen.

Geeignete Acrylat-Schreibmonomere sind insbesondere Verbindungen der allgemeinen Formel (II)

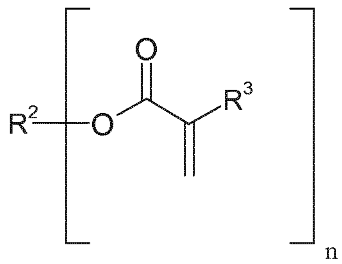

(II)

bei denen n≥1 und n≤4 ist und $R^2$ ein linearer, verzweigter, cyclischer oder heterocyclischer unsubstituierter oder gegebenenfalls auch mit Heteroatomen substituierter organischer Rest und/oder $R^3$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R^3$ Wasserstoff oder Methyl und/oder $R^2$ ein linearer, verzweigter, cyclischer oder heterocyclischer unsubstituierter oder gegebenenfalls auch mit Heteroatomen substituierter organischer Rest.

Als Acrylate bzw. Methacrylate werden vorliegend Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen, N-Carbazolylacrylate.

Als Urethanacrylate werden vorliegend Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanat-funktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri- oder Polyisocyanate sind oben genannt sowie Triphenylmethan-4,4',4"-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)-acrylate, Poly(ε-caprolacton)mono(meth)acrylate, wie z.B. Tone® M100 (Dow, Schwalbach, DE), 2-Hydroxypropyl(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)acrylat.

Ebenso ist es möglich, dass das Schreibmonomer weitere ungesättigte Verbindungen wie α,β-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol und / oder Olefine, umfasst.

Erfindungsgemäß geeignete Photoinitiatoren sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Farbstoff und Coinitiator der Typ II-Photoinitiatoren können entweder unmittelbar gemeinsam mit den weiteren Komponenten des Photopolymers vermischt werden oder aber auch jeweils mit Einzelkomponenten vorvermischt werden. Insbesondere wenn das Photopolymer Polyurethan-Matrixpolymere enthalten soll, kann der Farbstoff mit der Isocyanat-reaktiven Komponente und der Coinitiator mit der Isocyanat-Komponente vorvermischt werden. Ebenso ist es aber auch möglich den Coinitiator mit der Isocyanat-reaktiven Komponente und den Farbstoff mit der Isocyanat-Komponente vorzuvermischen.

Solche Photoinitiatorsysteme sind prinzipiell in der EP 0 223 587 A beschriebenen und bestehen bevorzugt aus einer Mischung von einem oder mehreren Farbstoffen mit Ammoniumalkylarylborat(en).

Geeignete Farbstoffe, die zusammen mit einem Ammoniumalkylarylborat einen Typ II-Photoinitiator bilden, sind die in der WO 2012062655 beschriebenen kationischen Farbstoffe in Kombination mit den eben dort beschriebenen Anionen.

Unter kationischen Farbstoffen werden bevorzugt solche der folgenden Klassen verstanden: Acridin-Farbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)pyrylium, Safranin O, Astraphloxin, Brilliant Grün, Kristallviolett, Ethylviolett und Thionin.

Bevorzugte Anionen sind insbesondere $C_8$- bis $C_{25}$-Alkansulfonat, vorzugsweise $C_{13}$- bis $C_{25}$-Alkansulfonat, $C_3$- bis $C_{18}$-Perfluoralkansulfonat, $C_4$- bis $C_{18}$-Perfluoralkansulfonat, das in der Alkylkette mindestens 3 Wasserstoffatome trägt, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_3$- bis $C_{18}$-Perfluoralkylsulfat, $C_4$-bis $C_{18}$-Perfluoralkylsulfat, das in der Alkylkette mindestens 3 Wasserstoffatome trägt, Polyethersulfate basierend auf mindestens 4 Äquivalenten Ethylenoxid und/oder Äquivalenten 4 Propylenoxid, Bis-$C_4$- bis $C_{25}$-Alkyl-, $C_5$- bis $C_7$-Cycloalkyl-, $C_3$- bis Cs-Alkenyl- oder $C_7$-bis $C_{11}$-Aralkyl-sulfosuccinat, durch mindestens 8 Fluoratome substituiertes Bis-$C_2$-bis $C_{10}$-alkylsulfosuccinat, $C_8$- bis $C_{25}$-Alkyl-sulfoacetate, durch mindestens einen Rest der Gruppe Halogen, $C_4$- bis $C_{25}$-Alkyl, Perfluor-$C_1$- bis Cs-Alkyl und/oder $C_1$- bis $C_{12}$-Alkoxycarbonyl substituiertes Benzolsulfonat, ggf. durch Nitro, Cyano, Hydroxy, $C_1$- bis $C_{25}$-Alkyl, $C_1$- bis $C_{12}$-Alkoxy, Amino, $C_1$- bis $C_{12}$-Alkoxycarbonyl oder Chlor substituiertes Naphthalin- oder Biphenylsulfonat, ggf. durch Nitro, Cyano, Hydroxy, $C_1$- bis $C_{25}$-Alkyl, $C_1$- bis $C_{12}$-Alkoxy, $C_1$- bis $C_{12}$-Alkoxycarbonyl oder Chlor substituiertes Benzol-, Naphthalin- oder Biphenyldisulfonat, durch Dinitro, $C_6$- bis $C_{25}$-Alkyl, $C_4$- bis $C_{12}$-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$- bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, $C_4$- bis $C_{12}$-Alkyl-triphenylborat, deren Phenyl- oder Phenoxy-Reste durch Halogen, $C_1$- bis $C_4$-Alkyl und/oder $C_1$- bis $C_4$-Alkoxy substituiert sein können, $C_4$- bis $C_{12}$-Alkyl-trinaphthylborat, Tetra-Ci- bis $C_{20}$-alkoxyborat, 7,8- oder 7,9-Dicarba-nido-undecaborat(1-) oder (2-), die gegebenenfalls an den B- und/oder C-Atomen durch eine oder zwei $C_1$- bis $C_{12}$-Alkyl- oder Phenyl-Gruppen substituiert sind, Dodecahydro-dicarbadodecaborat(2-) oder B-$C_1$- bis $C_{12}$-Alkyl-C-phenyl-dodecahydro-dicarbadodecaborat(1-) steht, wobei bei mehrwertigen Anionen wie Naphthalindisulfonat $A^-$ für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl substituiert sein können.

Bevorzugt ist auch, wenn das Anion $A^-$ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Geeignete Ammoniumalkylarylborate sind beispielsweise (Cunningham et al., RadTech'98 North America UV/EB Conference Proceedings, Chicago, Apr. 19-22, 1998): Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat hexylborat ([191726-69-9], CGI 7460, Produkt der BASF SE, Basel, Schweiz), 1-Methyl-3-octylimidazolium Dipentyldiphenylborat und Tetrabutylammonium Tris-(3-chlor-4-methylphenyl)-hexylborat ([1147315-11-4], CGI 909, Produkt der BASF SE, Basel, Schweiz).

Es kann vorteilhaft sein, Gemische dieser Photoinitiatoren einzusetzen. Je nach verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Ganz besonders bevorzugt ist, wenn der Photoinitiator eine Kombination von Farbstoffen, deren Absorptionsspektren zumindest teilweise den Spektralbereich von 400 bis 800 nm abdecken, mit wenigstens einem auf die Farbstoffe abgestimmten Coinitiator umfasst.

Bevorzugt ist auch, wenn wenigstens ein für eine Laserlichtfarbe ausgewählt aus blau, grün und rot geeigneter Photoinitiator in der Photopolymer-Formulierung enthalten ist.

Weiter bevorzugt ist auch, wenn die Photopolymer-Formulierung für wenigstens zwei Laserlichtfarben ausgewählt aus blau, grün und rot je einen geeigneten Photoinitiator enthält.

Ganz besonders bevorzugt ist schließlich, wenn die Photopolymer-Formulierung für jede der Laserlichtfarben blau, grün und rot jeweils einen geeigneten Photoinitiator enthält.

Gemäß einer weiteren bevorzugten Ausführungsform ist vorgesehen, dass die Photopolymer-Formulierung zusätzlich monomere Urethane als Additive enthält, wobei die Urethane insbesondere mit wenigstens einem Fluoratom substituiert sein können.

Bevorzugt können die Urethane die allgemeine Formel (III)

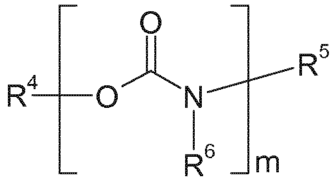

(III)

haben, in der m≥1 und m≤8 ist und $R^4$, $R^5$ und $R^6$ lineare, verzweigte, cyclische oder heterocyclische unsubstituierte oder gegebenenfalls auch mit Heteroatomen substituierte organische Reste und/oder $R^5$, $R^6$ unabhängig voneinander Wasserstoff sind, wobei bevorzugt mindestens einer der Reste $R^4$, $R^5$, $R^6$ mit wenigstens einem Fluoratom substituiert ist und besonders bevorzugt $R^4$ ein organischer Rest mit mindestens einem Fluoratom ist. Besonders bevorzugt ist $R^5$ ein linearer, verzweigter, cyclischer oder heterocyclischer unsubstituierter oder gegebenenfalls auch mit Heteroatomen wie beispielsweise Fluor substituierter organischer Rest.

Ebenfalls Gegenstand der Erfindung ist ein holografisches Medium, umfassend Matrixpolymere, Schreibmonomere und Photoinitiatoren, wobei die Schreibmonomere eine erfindungsgemäße Verbindung gemäß Formel (I) umfassen.

Die für die erfindungsgemäße Photopolymer-Formulierung beschriebenen, oben stehenden, weiteren bevorzugten Ausführungsformen auch bevorzugte Ausführungsformen des erfindungsgemäßen holographischen Mediums dar.

Gemäß einer bevorzugten Ausführungsform des holgraphischen Mediums ist vorgesehen, dass die Matrixpolymere vernetzt, bevorzugt dreidimensional vernetzt und ganz besonders bevorzugt dreidimensional vernetzte Polyurethane sind.

Bevorzugt ist auch, wenn das holographische Medium wenigstens ein Fluorurethan als Additiv umfasst.

Das holografische Medium kann insbesondere ein Films sein, der bevorzugt eine Filmdicke von 0.5 μm bis 200 μm, weiter bevorzugt von 0.8 μm bis 50 μm und besonders bevorzugt von 1μm bis 25 μm aufweisen kann.

In das holografische Medium kann auch wenigstens ein Hologramm einbelichtet sein.

In die erfindungsgemäßen holographischen Medien können durch entsprechende Belichtungsprozesse für optische Anwendungen im gesamten sichtbaren und nahen UV-Bereich (300-800 nm) Hologrammen einbelichtet werden. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflektionshologramme, Denisyukhologramme, Transmissionshologramme.

Mögliche optische Funktionen der Hologramme entsprechen den optische Funktionen von Lichtelementen wie Linsen, Spiegel, Umlenkspiegel, Filter, Streuscheiben, Beugungselemente, Diffusoren, Lichtleiter, Lichtlenker (waveguides), Projektionsscheiben und/oder Masken. Ebenfalls können Kombinationen aus dies optischen Funktionen unabhängig voneinander in einem Hologramm vereinigt sein. Häufig zeigen diese optischen Elemente eine Frequenzselektivität, je nachdem wie die Hologramme belichtet wurden und welche Dimensionen das Hologramm hat.

Zudem können mittels der erfindungsgemäßen Medien auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Design-Möglichkeiten stellen Hologramme, insbesondere Volumenhologramme, eine attraktive technische Lösung für die oben genannten Anwendung dar.

Weiterhin Gegenstand der vorliegenden Erfindung ist die Verwendung eines erfindungsgemäßen holographischen Mediums zur Aufzeichnung von In-Line, Off-Axis, Full-Aperture Transfer, Weißlicht-Transmissions, Denisyuk, Off-Axis Reflektions oder Edge-Lit Hologrammen sowie holographischen Stereogrammen, insbesondere zur Herstellung von optischen Elementen, Bildern oder Bilddarstellungen.

Ebenfalls Gegenstand der vorliegenden Erfindung ist ein Verfahren zur Herstellung eines holographischen Mediums unter Verwendung einer erfindungsgemäßen Photopolymer-Formulierung.

Die Photopolymer-Formulierungen können insbesondere zur Herstellung holographischer Medien in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

Noch ein Gegenstand der Erfindung ist auch ein Display umfassend ein erfindungsgemäßes holografische Medium.

Beispiele für derartige Displays sind beispielsweise in der US 2009/174919 A1, WO 2012/035058 und in der nicht veröffentlichten Anmeldung EP 14155599.5 beschrieben.

Die vorliegende Erfindung wird im Folgenden anhand von Beispielen näher erläutert.

**Messmethoden:**

| | |
|---|---|
| OH Zahl: | Die angegebenen OH-Zahlen wurden gemäß DIN 53240-2 bestimmt. |
| NCO-Wert: | Die angegebenen NCO-Werte (Isocyanat-Gehalte) wurden gemäß DIN EN ISO 11909 bestimmt. |
| Festkörpergehalt: | Die angegebenen Festkörpergehalte wurden gemäß DIN EN ISO 3251 bestimmt. |

Messung der holographischen Eigenschaften DE und $\Delta n$ der holographischen Medien mittels Zweistrahlinterferenz in Reflexionsanordnung

Die wie im Abschnitt "Herstellung der Medien zur Bestimmung der holographischen Eigenschaften" beschrieben hergestellten Medien wurden mittels einer Messanordnung gemäß Figur 1 wie folgt auf ihre holographischen Eigenschaften geprüft: Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel ($\alpha_0$) des Referenzstrahls beträgt - 21.8°, der Einfallswinkel ($\beta_0$) des Signalstrahls beträgt 41.8°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand Λ, auch Gitterperiode genannt, im Medium beträgt ~ 225 nm (der Brechungsindex des Mediums zu ~1.504 angenommen).

Figur 1 zeigt die Geometrie eines Holographic Media Testers (HMT) bei $\lambda$ = 633 nm (He-Ne Laser): M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -21.8°, $\beta_0$ = 41.8° sind die Einfallswinkel der kohärenten Strahlen außerhalb der Probe (des Mediums) gemessen. RD = Referenzrichtung des Drehtisches. Mit einem holographischen Versuchsaufbau wie in Figur 1 dargestellt wurden die Beugungseffizienz (DE) der Medien gemessen.

Es wurden auf folgende Weise Hologramme in das Medium geschrieben:

- Beide Shutter (S) sind für die Belichtungszeit t geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls

blieb geschlossen. Der Shutter des Referenzstrahls war geöffnet. Die Irisblende des Referenzstrahls wurde auf einen Durchmesser < 1mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $Q_{min}$ bis $Q_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording} .$$

$\theta_0$ ist der Halbwinkel im Laborsystem außerhalb des Mediums und es gilt beim Schreiben des Hologramms:

$$\theta_0 = \frac{\alpha_0 - \beta_0}{2} .$$

In diesem Fall gilt also $\theta_0$ = -31.8°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$, des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$ ermittelt. Eventuell musste dazu die Position des Detektors des abgebeugten Strahls verändert werden, um diesen maximalen Wert zu bestimmen.

Der Brechungsindexkontrast $\Delta$n und die Dicke *d* der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand $\Lambda'$ des Hologramms und die Orientierung der Streifen (slant) vom Streifenabstand $\Lambda$ des Interferenzmusters und dessen Orientierung abweichen kann. Demnach wird auch der Winkel $\alpha_0'$ bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve $\eta(\Omega)$ eines Reflexionshologramms gilt nach Kogelnik:

$$\eta = \begin{cases} \dfrac{1}{1-\dfrac{1-(\xi/\nu)^2}{\sin^2\left(\sqrt{\xi^2-\nu^2}\right)}}, \text{ für } \nu^2-\xi^2<0 \\ \dfrac{1}{1+\dfrac{1-(\xi/\nu)^2}{\sinh^2\left(\sqrt{\nu^2-\xi^2}\right)}}, \text{ für } \nu^2-\xi^2\geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left( 2 \cdot \cos(\psi' - \vartheta') - \frac{\lambda}{n \cdot \Lambda'} \right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$

$$\sin(\vartheta'_0) = n \cdot \sin(\vartheta')$$

An der Bragg-Bedingung ist das "Dephasing" *DP* = 0. Und es folgt entsprechend:

$$\alpha'_0 = \theta_0 + \Omega_{reconstruction}$$

$$\sin(\alpha'_0) = n \cdot \sin(\alpha')$$

Der noch unbekannt Winkel $\beta'$ kann aus dem Vergleich der Bragg-Bedingung des Interferenzfeldes beim Schreiben des Hologramms und der Bragg-Bedingung beim Auslesen des Hologramms ermittelt werden unter der Annahme,

dass nur Dickenschwindung stattfindet. Dann folgt:

$$\sin(\beta') = \frac{1}{n} \cdot \left[\sin(\alpha_0) + \sin(\beta_0) - \sin(\theta_0 + \Omega_{reconstruction})\right]$$

v ist die Gitterstärke, ξ ist der Detuning Parameter und ψ' die Orientierung (Slant) des Brechungsindexgitters das geschrieben wurde. α' und β' entsprechen den Winkeln $\alpha_0$ und $\beta_0$ des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. λ ist die Wellenlänge des Laserlichts im Vakuum.

Die maximale Beugungseffizienz (DE = $\eta_{max}$) ergibt sich dann für ξ = 0 zu:

$$DE = \tanh^2(\nu) = \tanh^2\left(\frac{\pi \cdot \Delta n \cdot d'}{\lambda \cdot \sqrt{\cos(\alpha') \cdot \cos(\alpha' - 2\psi)}}\right)$$

Figur 2 zeigt die gemessene transmittierte Leistung $P_T$ (rechte *y*-Achse) als durchgezogene Linie gegen das Winkeldetuning ΔΩ aufgetragen, die gemessene Beugungseffizienz η (linke *y*-Achse) als ausgefüllte Kreise gegen das Winkeldetuning ΔΩ aufgetragen (soweit die endliche Größe des Detektors es erlaubte) und die Anpassung der Kogelnik Theorie als gestrichelte Linie (linke *y*-Achse).

Die Messdaten der Beugungseffizienz, die theoretische Braggkurve und die transmittierte Intensität werden wie in Figur 2 gezeigt gegen den zentrierten Drehwinkel $\Delta\Omega \equiv \Omega_{reconstruction} - \Omega = \alpha'_0 - \vartheta'_0$, auch Winkeldetuning genannt, aufgetragen.

Da DE bekannt ist wird die Form der theoretischen Braggkurve nach Kogelnik nur noch durch die Dicke d' der Photopolymerschicht bestimmt. Δn wird über DE für gegebene Dicke *d'* so nachkorrigiert, dass Messung und Theorie von DE immer übereinstimmen. *d'* wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines Ω-Scans mitrotiert, der Detektor für das abgebeugte Licht aber nur einen endlichen Winkelbereich erfassen kann, wird die Braggkurve von breiten Holgrammen (kleines *d'*) bei einem Ω-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke d' zusätzlich herangezogen.

Figur 2 zeigt die Darstellung der Braggkurve η nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning ΔΩ.

Für eine Formulierung wurde diese Prozedur eventuell mehrfach für verschiedene Belichtungszeiten *t* an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis E ergibt sich wie folgt aus den Leistungen der zwei den Winkeln $\alpha_0$ und $\beta_0$ zugeordneten Teilstrahlen (Referenzstrahl mit $P_r$ = 0.50 mW und Signalstrahl mit $P_s$ = 0.63 mW), der Belichtungszeit t und dem Durchmesser der Irisblende (0.4 cm):

$$E\ (\mathrm{mJ/cm^2}) = \frac{2 \cdot [P_r + P_s] \cdot t\ (\mathrm{s})}{\pi \cdot 0.4^2\ \mathrm{cm}^2}$$

Die Leistungen der Teilstrahlen wurden so angepasst, dass in dem Medium bei den verwendeten Winkeln ao und $\beta_0$, die gleiche Leistungsdichte erreicht wird.

**Substanzen:**

Die verwendeten Lösungsmittel und Reagenzien wurden im Chemikalienhandel bezogen.

| | |
|---|---|
| BINOL | (+/-)-1,1'-Bi(2-naphthol) [602-09-5] ist bei der ABCR GmbH & Co. KG, Karlsruhe, Deutschland erhältlich. |

(fortgesetzt)

| | |
|---|---|
| 6,6'-Dibromo-1,1'-bi-naphthalen-2,2'-diol | [80655-81-8], ist bei der ABCR GmbH & Co. KG, Karlsruhe, Deutschland erhältlich. |
| 7-Methoxy-2-naphthol | [5060-82-2] ist bei der Aldrich Chemie, Steinheim, Deutschland erhältlich. |
| Methyl-3-hydroxy-2-naphthoat | [883-99-8] ist bei der ABCR GmbH & Co. KG, Karlsruhe, Deutschland erhältlich. |
| 6-Bromo-2-naphthol | [15231-91-1] ist bei der ABCR GmbH & Co. KG, Karlsruhe, Deutschland erhältlich. |
| 6-Cyan-2-naphthol | [52927-22-7] ist bei der ABCR GmbH & Co. KG, Karlsruhe, Deutschland erhältlich. |
| 2-(Phenylthio)-phenylisocyanat | [13739-55-4], ist bei der ABCR GmbH & Co. KG, Karlsruhe, Deutschland erhältlich. |
| CGI-909 | Tetrabutylammonium-tris(3-chlor-4-methylphenyl)(hexyl)borat, [1147315-11-4] ist ein von der CIBA Inc., Basel, Schweiz, hergestelltes Produkt. |
| Desmodur® N 3900 | Produkt der Bayer MaterialScience AG, Leverkusen, DE, Hexandiisocyanat-basiertes Polyisocyanat, Anteil an Iminooxadiazindion mindestens 30 %, NCO-Gehalt: 23.5 %. |
| Desmodur® H | Produkt der Bayer MaterialScience AG, Leverkusen, DE, monomeres aliphatisches Diisocyanat, Hexamethylen-1,6-diisocyanat (HDI) oder 1,6-Diisocyanatohexan, NCO-Gehalt: ≥49.7 %. |
| Vestanat TMDI | Produkt der Evonik Industries AG, Essen, DE, monomeres substituiertes aliphatisches Diisocyanat, ein ca. 1:1 Gemisch von 2,2,4-und 2,4,4-Trimethyl-hexamethylendiisocyanat. Diesem Gemisch wurde bei der Bezeichnung der hergestellten Beispiele aus Gründen der Übersichtlichkeit nicht Rechnung getragen und nur das jeweilige 2,2,4-Isomer beschrieben. Das ebenfalls entstehende 2,4,4-Isomer ist ebenfalls gemeint und umfasst. |
| Desmorapid Z | Dibutylzinn-dilaurat [77-58-7], Produkt der Bayer MaterialScience AG, Leverkusen, Deutschland. |
| Fomrez UL 28 | Urethanisierungskatalysator, Produkt der Momentive Performance Chemicals, Wilton, CT, USA. |
| Borchi® Kat 22 | Urethanisierungskatalysator, Produkt der OMG Borchers GmbH, Langenfeld, Deutschland. |
| KarenzAOI® | 2-Isocyanatoethylacrylat, [13641-96-8], Produkt der SHOWA DENKO K.K., Fine Chemicals Group, Specialty Chemicals Department, Chemicals Division. |
| KarenzMOI® | 2-Isocyanatoethyl-methacrylat, [30674-80-7], Produkt der SHOWA DENKO K.K., Fine Chemicals Group, Specialty Chemicals Department, Chemicals Division. |
| Farbstoff 1 | C. I. Basic Blue 3 (als Bis-(2-ethylhexyl)sulfosuccinat) wurde nach dem aus WO2012062655 bekannten Verfahren hergestellt, Beispiel 9. |

Sofern nicht abweichend vermerkt beziehen sich alle Prozentangaben auf Gewichtsprozent.

**Beispiel 1: 2-[({[2'-({[2-(Acryloyloxy)ethyl]carbamoyl}oxy)-1,1'-binaphthyl-2-yl]oxy}carbonyl)amino]ethyl methacrylat**

In einem 100 mL Rundkolben wurden 18.4 g BINOL, 0.08 g Desmorapid Z und 0.03 g 2,6-Di-tert.-butyl-4-methylphenol in 25 mL Dichlormethan vorgelegt. Anschließend wurde eine 1:1 Mischung von 10.0 g KarenzMOI® und 9.1 g KarenzAOI® zugetropft und die Mischung bei Raumtemperatur gerührt, bis der Isocyanatgehalt unter 0.1 % gesunken war. Anschließend wurde das Produkt im Rotationsverdampfer vom Dichlormethan befreit. Das Produkt wurde als farbloser Feststoff erhalten.

**Beispiel 2: Dimethyl 2,2'-bis({[2-(methacryloyloxy)ethyl]carbamoyl}oxy)-1,1'-binaphthyl-3,3'-dicarboxylat**

In einem 25 mL Rundkolben wurden 3.0 g Dimethyl 2,2'-dihydroxy-1,1'-binaphthyl-3,3'-dicarboxylat ([47644-69-9], hergestellt wie in Tetrahedron Letters (1994), 35(43), 7983-4 beschrieben), 0.01 g Borchi® Kat 22 und 0.01 g 2,6-Di-tert.-butyl-4-methylphenol in 10 mL Ethylacetat vorgelegt. Anschließend wurden 2.3 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 3: 1,1'-Binaphthyl-2,2'-diylbis(oxycarbonyliminoethan-2,1-diyl)bisacrylat**

In einem 250 mL Rundkolben wurden 40.0 g BINOL, 0.18 g Desmorapid Z und 0.06 g 2,6-Di-tert.-butyl-4-methylphenol in 80 mL Dichlormethan vorgelegt. Anschließend wurden 39.4 g KarenzAOI® zugetropft und die Mischung bei Raumtemperatur gerührt, bis der Isocyanatgehalt unter 0.1 % gesunken war. Anschließend wurde das Produkt im Rotationsverdampfer vom Dichlormethan befreit. Das Produkt wurde als farbloser Feststoff erhalten.

**Beispiel 4: 1,1'-Binaphthyl-2,2'-diylbis(oxycarbonyliminoethan-2,1-diyl)bis(2-methylacrylat)**

In einem 100 mL Rundkolben wurden 12.0 g BINOL, 0.05 g Desmorapid Z und 0.02 g 2,6-Di-tert.-butyl-4-methylphenol in 25 mL Dichlormethan vorgelegt. Anschließend wurden 13.0 g KarenzMOI® zugetropft und die Mischung bei Raumtemperatur gerührt, bis der Isocyanatgehalt unter 0.1 % gesunken war. Anschließend wurde das Produkt im Rotationsverdampfer vom Dichlormethan befreit. Das Produkt wurde als farbloser Feststoff erhalten.

**Beispiel 5: (6,6'-Dicyano-1,1'-binaphthyl-2,2'-diyl)bis(oxycarbonyliminoethan-2,1-diyl) bisacrylat**

In einem 25 mL Rundkolben wurden 3.0 g 2,2'-Dihydroxy-1,1'-binaphthyl-6,6'-dicarbonitril ([164171-19-1], hergestellt aus 6-Cyan-2-naphthol wie in Tetrahedron Letters (1994), 35(43), 7983-4 beschrieben), 0.01 g Borchi® Kat 22 und 0.01 g 2,6-Di-tert.-butyl-4-methylphenol in 10 mL Ethylacetat vorgelegt. Anschließend wurden 2.5 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 6: (6,6'-Dibromo-1,1'-binaphthyl-2,2'-diyl)bis(oxycarbonyliminoethan-2,1-diyl) bisacrylat**

In einem 25 mL Rundkolben wurden 2.5 g 6,6'-Dibromo-1,1'-binaphthyl-2,2'-diol ([80655-81-8], erhältlich von ABCR GmbH & Co. KG, Karlsruhe, Deutschland), 0.01 g Borchi® Kat 22 und 0.01 g 2,6-Di-tert.-butyl-4-methylphenol in 10 mL Ethylacetat vorgelegt. Anschließend wurden 1.6 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 7: (6,6'-Dibromo-1,1'-binaphthyl-2,2'-diyl)bis(oxycarbonyliminoethan-2,1-diyl) bis(2-methylacrylat)**

In einem 25 mL Rundkolben wurden 2.5 g 6,6'-Dibromo-1,1'-binaphthyl-2,2'-diol ([80655-81-8], erhältlich von ABCR GmbH & Co. KG, Karlsruhe, Deutschland), 0.01 g Borchi® Kat 22 und 0.01 g 2,6-Di-tert.-butyl-4-methylphenol in 10 mL Ethylacetat vorgelegt. Anschließend wurden 1.7 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Referenzbeispiel 8. (7,7'-Dimethoxy-1,1'-binaphthyl-2,2'-diyl)bis(oxycarbonyliminoethan-2,1-diyl) bisacrylat**

In einem 25 mL Rundkolben wurden 3.0 g 7,7'-Dimethoxy-1,1'-binaphthyl-2,2'-diol ([128702-28-3], hergestellt aus 7-Methoxy-2-naphthol wie in Tetrahedron Letters (1994), 35(43), 7983-4 beschrieben), 0.01 g Borchi® Kat 22 und 0.01 g 2,6-Di-tert.-butyl-4-methylphenol in 10 mL Ethylacetat vorgelegt. Anschließend wurden 2.5 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 9: 2-{[({2'-[(Hexylcarbamoyl)oxy]-1,1'-binaphthyl-2-yl}oxy)carbonyl]amino}ethyl acrylat**

In einem 250 mL Rundkolben wurden 34.6 g BINOL und 0.09 g Borchi® Kat 22 in 50 mL Ethylacetat vorgelegt. Anschließend wurden 15.4 g Hexylisocyanat zugetropft und die Mischung bei 80 °C gerührt, bis der Isocyanatgehalt unter 0.1 % gesunken war. Anschließend wurde das Produkt im Rotationsverdampfer vom Ethylacetat befreit. Es wurden 49.3 g 2'-Hydroxy-1,1'-binaphthyl-2-yl-hexylcarbamat als farbloser Feststoff erhalten.

In einem 100 mL Rundkolben wurden 18.6 g 2'-Hydroxy-1,1'-binaphthyl-2-yl-hexylcarbamat, 0.03 g Borchi® Kat 22 und 0.01 g 2,6-Di-tert.-butyl-4-methylphenol in 25 mL Ethylacetat vorgelegt. Anschließend wurden 6.35 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 10: 2-{[({2'-[(Hexylcarbamoyl)oxy]-1,1'-binaphthyl-2-yl}oxy)carbonyl]amino}ethyl methacrylat**

In einem 100 mL Rundkolben wurden 18.1 g 2'-Hydroxy-1,1'-binaphthyl-2-yl-hexylcarbamat (siehe Beispiel 9), 0.03 g Borchi® Kat 22 und 0.01 g 2,6-Di-tert.-butyl-4-methylphenol in 25 mL Ethylacetat vorgelegt. Anschließend wurden 6.81 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel** 11: **2-{[({2'-[(Hexylcarbamoyl)oxy]-1,1'-binaphthyl-2-yl}oxy)carbonyl]amino}ethyl acrylat**

In einem 250 mL Rundkolben wurden 35.3 g BINOL und 0.09 g Borchi® Kat 22 in 50 mL Ethylacetat vorgelegt. Anschließend wurden 14.7 g Phenylisocyanat zugetropft und die Mischung bei 80 °C gerührt, bis der Isocyanatgehalt unter 0.1 % gesunken war. Anschließend wurde das Produkt im Rotationsverdampfer vom Ethylacetat befreit. Es wurden 49.2 g 2'-Hydroxy-1,1'-binaphthyl-2-yl-phenylcarbamat als farbloser Feststoff erhalten.

In einem 100 mL Rundkolben wurden 18.5 g 2'-Hydroxy-1,1'-binaphthyl-2-yl-phenylcarbamat, 0.03 g Borchi® Kat 22 und 0.01 g 2,6-Di-tert.-butyl-4-methylphenol in 25 mL Ethylacetat vorgelegt. Anschließend wurden 6.44 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 12: 2-{[({2'-[(Hexylcarbamoyl)oxy]-1,1'-binaphthyl-2-yl}oxy)carbonyl]amino}ethyl acrylat**

In einem 100 mL Rundkolben wurden 18.0 g 2'-Hydroxy-1,1'-binaphthyl-2-yl-phenylcarbamat (siehe Beispiel 11), 0.03 g Borchi® Kat 22 und 0.01 g 2,6-Di-tert.-butyl-4-methylphenol in 25 mL Ethylacetat vorgelegt. Anschließend wurden 6.90 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 13: 2-[({[2'-({[3-(Methylsulfanyl)phenyl]carbamoyl}oxy)-1,1'-binaphthyl-2-yl]oxy}carbonyl)amino]ethyl acrylat**

In einem 250 mL Rundkolben wurden 31.7 g BINOL und 0.08 g Borchi® Kat 22 in 50 mL Ethylacetat vorgelegt. Anschließend wurden 18.3 g 3-Methylthio-phenylisocyanat zugetropft und die Mischung bei 80 °C gerührt, bis der Isocyanatgehalt unter 0.1 % gesunken war. Anschließend wurde das Produkt im Rotationsverdampfer vom Ethylacetat befreit. Es wurden 49.5 g 2'-Hydroxy-1,1'-binaphthyl-2-yl [3-(methylsulfanyl)phenyl]carbamat als farbloser Feststoff erhalten.

In einem 100 mL Rundkolben wurden 19.0 g 2'-Hydroxy-1,1'-binaphthyl-2-yl [3-(methylsulfanyl)phenyl]carbamat, 0.03 g Borchi® Kat 22 und 0.02 g 2,6-Di-tert.-butyl-4-methylphenol in 25 mL Ethylacetatvorgelegt. Anschließend wurden 5.94 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 14: 2-{[({2'-[(Hexylcarbamoyl)oxy]-1,1'-binaphthyl-2-yl}oxy)carbonyl]amino}ethyl methacrylat**

In einem 100 mL Rundkolben wurden 18.6 g 2'-Hydroxy-1,1'-binaphthyl-2-yl [3-(methylsulfanyl)phenyl]carbamat (siehe Beispiel 13), 0.03 g Borchi® Kat 22 und 0.01 g 2,6-Di-tert.-butyl-4-methylphenol in 25 mL Ethylacetat vorgelegt. Anschließend wurden 6.38 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel15: 2-[({[2'-({[3-(Methylsulfanyl)phenyl]carbamoyl}oxy)-1,1'-binaphthyl-2-yl]oxy}carbonyl)amino]ethyl acrylat**

In einem 250 mL Rundkolben wurden 27.8 g BINOL und 0.07 g Borchi® Kat 22 in 50 mL Ethylacetat vorgelegt. Anschließend wurden 22.1 g 2-Isocyanatophenyl-phenyl-sulfid ([13739-55-4] zugetropft und die Mischung bei 80 °C gerührt, bis der Isocyanatgehalt unter 0.1 % gesunken war. Anschließend wurde das Produkt im Rotationsverdampfer vom Ethylacetat befreit. Es wurden 48.9 g 2'-Hydroxy-1,1'-binaphthyl-2-yl [2-(phenylsulfanyl)-phenyl]carbamat als farbloser Feststoff erhalten.

In einem 100 mL Rundkolben wurden 19.6 g 2'-Hydroxy-1,1'-binaphthyl-2-yl [2-(phenylsulfanyl)phenyl]carbamat, 0.03 g Borchi® Kat 22 und 0.02 g 2,6-Di-tert.-butyl-4-methylphenol in 25 mL Ethylacetatvorgelegt. Anschließend wurden 5.38 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 16: 2-{[({2'-[(Hexylcarbamoyl)oxy]-1,1'-binaphthyl-2-yl}oxy)carbonyl]amino}ethyl methacrylat**

In einem 100 mL Rundkolben wurden 19.2 g 2'-Hydroxy-1,1'-binaphthyl-2-yl [2-(phenylsulfanyl)phenyl]carbamat (siehe Beispiel 15), 0.03 g Borchi® Kat 22 und 0.01 g 2,6-Di-tert.-butyl-4-methylphenol in 25 mL Ethylacetat vorgelegt. Anschließend wurden 5.79 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 17: 2-{[({2'-[(1-Naphthylcarbamoyl)oxy]-1,1'-binaphthyl-2-yl}oxy)carbonyl]amino}ethyl acrylat**

In einem 250 mL Rundkolben wurden 31.4 g BINOL und 0.08 g Borchi® Kat 22 in 50 mL Ethylacetat vorgelegt. Anschließend wurden 18.5 g 1-Naphtylisocyanat zugetropft und die Mischung bei 80 °C gerührt, bis der Isocyanatgehalt unter 0.1 % gesunken war. Anschließend wurde das Produkt im Rotationsverdampfer vom Ethylacetat befreit. Es wurden 49.1 g 2'-Hydroxy-1,1'-binaphthyl-2-yl 1-naphthylcarbamat als farbloser Feststoff erhalten.

In einem 100 mL Rundkolben wurden 19.0 g 2'-Hydroxy-1,1'-binaphthyl-2-yl [2-(phenylsulfanyl)phenyl]carbamat, 0.03 g Borchi® Kat 22 und 0.02 g 2,6-Di-tert.-butyl-4-methylphenol in 25 mL Ethylacetatvorgelegt. Anschließend wurden 5.90 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 18: 2-{[({2'-[(1-Naphthylcarbamoyl)oxy]-1,1'-binaphthyl-2-yl}oxy)carbonyl]amino}ethyl methacrylat**

In einem 100 mL Rundkolben wurden 18.6 g 2'-Hydroxy-1,1'-binaphthyl-2-yl 1-naphthylcarbamat (siehe Beispiel 17), 0.03 g Borchi® Kat 22 und 0.01 g 2,6-Di-tert.-butyl-4-methylphenol in 25 mL Ethylacetat vorgelegt. Anschließend wurden 6.34 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 19: Hexan-1,6-diylbis(carbamoyloxy-1,1'-binaphthyl-2',2-diyloxycarbonyliminoethan-2,1-diyl) bisacrylat**

In einem 250 mL Rundkolben wurden 37.2 g BINOL in 150 g Ethylacetat bei 80 °C vorgelegt und 0.005 g Desmorapid Z zugegeben. Unter intensivem Rühren wurden 10.7 g Hexamethylen-diisocyanat (Desmodur H, Produkt von Bayer MaterialScience AG, NCO-Gehalt >49.7%) zugegeben und solange bei dieser Temperatur gerührt, bis der Isocyanatgehalt unter 0.1 % gesunken war. Das so erhaltene Bis(2'-hydroxy-1,1'-binaphthyl-2-yl) hexan-1,6-diylbiscarbamat hatte einen Festkörpergehalt von 24.2% in Ethylacetat.

Zu 61.2g der obigen Lösung von Bis(2'-hydroxy-1,1'-binaphthyl-2-yl) hexan-1,6-diylbiscarbamat in Ethylacetat wurden unter Durchleitung von Luft bei 80 °C 5.6 g KarenzAOI® getropft und solange bei dieser Temperatur gerührt, bis der Isocyanatgehalt unter 0.1 % gesunken war. Anschließend wurde das Produkt im Rotationsverdampfer vom Ethylacetat befreit. Das Produkt wurde als farbloses Öl erhalten.

**Beispiel 20: Hexan-1,6-diylbis(carbamoyloxy-1,1'-binaphthyl-2',2-diyloxycarbonyliminoethan-2,1-diyl) bis(2-methylacrylat)**

Zu 61.2 g der Lösung von Bis(2'-hydroxy-1,1'-binaphthyl-2-yl) hexan-1,6-diylbiscarbamat in Ethylacetat (siehe Beispiel 19) wurden unter Durchleitung von Luft bei 80 °C 6.2 g KarenzMOI® getropft und solange bei dieser Temperatur gerührt, bis der Isocyanatgehalt unter 0.1 % gesunken war. Anschließend wurde das Produkt im Rotationsverdampfer vom Ethylacetat befreit. Das Produkt wurde als farbloses Öl erhalten.

**Beispiel 21: (2,2,4-Trimethylhexane-1,6-diyl)bis(carbamoyloxy-1,1'-binaphthyl-2',2-diyloxycarbonyliminoethan-2,1-diyl)- bisacrylat**

In einem 250 mL Rundkolben wurden 40.1 g BINOL in 150 g Ethylacetat bei 80 °C vorgelegt und 0.005 g

Desmorapid Z zugegeben. Unter intensivem Rühren wurden 14.5 g Trimethylhexamethylendiisocyanat (Vestanat TMDI, Produkt von Evonik Industries, NCO-Gehalt = 40.0%) zugegeben und solange bei dieser Temperatur gerührt, bis der Isocyanatgehalt unter 0.1 % gesunken war. Das so erhaltene Bis(2'-hydroxy-1,1'-binaphthyl-2-yl) (2,2,4-trimethylhexan-1,6-diyl)biscarbamat hatte einen Festkörpergehalt von 26.7% in Ethylacetat.

Zu 58.7 g der obigen Lösung von Bis(2'-hydroxy-1,1'-binaphthyl-2-yl) (2,2,4-trimethylhexan-1,6-diyl)biscarbamat in Ethylacetat wurden unter Durchleitung von Luft bei 80 °C 5.6 g KarenzAOI® getropft und solange bei dieser Temperatur gerührt, bis der Isocyanatgehalt unter 0.1 % gesunken war. Anschließend wurde das Produkt im Rotationsverdampfer vom Ethylacetat befreit. Das Produkt wurde als farbloses Öl erhalten.

**Beispiel 22: (2,2,4-Trimethylhexane-1,6-diyl)bis(carbamoyloxy-1,1'-binaphthyl-2',2-diyloxycarbonyliminoethan-2,1-diyl)-bis(2-methylacrylat)**

Zu 58.7 g der Lösung von Bis(2'-hydroxy-1,1'-binaphthyl-2-yl) (2,2,4-trimethylhexan-1,6-diyl)biscarbamat in Ethylacetat (siehe Beispiel 21) wurden unter Durchleitung von Luft bei 80 °C 6.2 g KarenzMOI® getropft und solange bei dieser Temperatur gerührt, bis der Iso- cyanatgehalt unter 0.1 % gesunken war. Anschließend wurde das Produkt im Rotationsverdampfer vom Ethylacetat befreit. Das Produkt wurde als farbloses Öl erhalten.

**Beispiel 23: 2-({[(2'-{[(3-{[({[2'-({[2-(Acryloyloxy)ethyl]carbamoyl}oxy)-1,1'-binaphthyl-2-yl]oxy}carbonyl)amino]methyl}-3,5,5-trimethylcyclohexyl)carbamoyl]-oxy}-1,1'-binaphthyl-2-yl)oxy]carbonyl}amino)ethyl acrylat**

In einem 250 mL Rundkolben wurden 40.1 g BINOL in 150 g Ethylacetat bei 80 °C vorgelegt und 0.005 g Desmorapid Z zugegeben. Unter intensivem Rühren wurden 15.2 g 3-Isocyanatomethyl-3,5,5-tiimethylcyclohexylisocyanat (Desmodur I, Isophorondiisocyanat (IPDI) Produkt von Bayer MaterialScience AG, NCO-Gehalt >37.5%) zugegeben und solange bei dieser Temperatur gerührt, bis der Isocyanatgehalt unter 0.1 % gesunken war. Das so erhaltene 2'-Hydroxy-1,1'-binaphthyl-2-yl {3-[({[(2'-hydroxy-1,1'-binaphthyl-2-yl)oxy]carbonyl}amino)methyl]-3,5,5-trimethylcyclohexyl}carbamat hatte einen Festkörpergehalt von 26.9% in Ethylacetat.

Zu 59.1 g der obigen Lösung von 2'-Hydroxy-1,1'-binaphthyl-2-yl{3-[({[(2'-hydroxy-1,1'-binaphthyl-2-yl)oxy]carbonyl}amino)methyl]-3,5,5-trimethylcyclohexyl}carbamat in Ethylacetat wurden unter Durchleitung von Luft bei 80 °C 5.6 g KarenzAOI® getropft und solange bei dieser Temperatur gerührt, bis der Isocyanatgehalt unter 0.1 % gesunken war. Anschließend wurde das Produkt im Rotationsverdampfer vom Ethylacetat befreit. Das Produkt wurde als farbloses Öl erhalten.

**Beispiel 24: 2-({[(2'-{[(3-{[({[2'-({[2-(Methacryloyloxy)ethyl]carbamoyl}oxy)-1,1'-binaphthyl-2-yl]oxy}carbonyl)amino]methyl}-3,5,5-trimethylcyclohexyl)carbamoyl]oxy}-1,1'-binaphthyl-2-yl)oxy]carbonyl}amino)ethyl methacrylat)**

Zu 59.1 g der Lösung von 2'-Hydroxy-1,1'-binaphthyl-2-yl{3-[({[(2'-hydroxy-1,1'-binaphthyl-2-yl)oxy]carbonyl}amino)methyl]-3,5,5-trimethylcyclohexyl} carbamat in Ethylacetat (siehe Beispiel 23) wurden unter Durchleitung von Luft bei 80 °C 6.2 g KarenzMOI® getropft und solange bei dieser Temperatur gerührt, bis der Isocyanatgehalt unter 0.1 % gesunken war. Anschließend wurde das Produkt im Rotationsverdampfer vom Ethylacetat befreit. Das Produkt wurde als farbloses Öl erhalten.

**Beispiel 25: (6-Brom-1,1'-binaphthyl-2,2'-diyl)bis(oxycarbonyliminoethan-2,1-diyl) bisacrylat**

In einem Becherglas wurden 16.0 g 2-Naphthol, 22.5 g 6-Brom-2-naphthol und 1.5 g CuCl(OH)*TMEDA (hergestellt wie in Tetrahedron Letters 1994 (35), 7983-7984 beschrieben) intensiv vermischt und dann für 120 min auf 100 °C erhitzt. Das erhaltene Gemisch wurde mit 200 mL 10%-iger Ammoniak-Lösung und zweimal mit 200 mL Wasser gewaschen, getrocknet und chromatographisch gereinigt. Es wurden 21.2 g 6-Bromo-1,1'-binaphthyl-2,2'-diol erhalten.

4.41 g 6-Brom-1,1'-binaphthyl-2,2'-diol wurden mit 0.015 g Desmorapid Z und 0.01 g 2,6-Di-tert.-butyl-4-methylphenol in 25 mL Ethylacetat vorgelegt. Anschließend wurden 3.41 g KarenzAOI® zugetropft und die Mischung bei 80 °C gerührt, bis der Isocyanatgehalt unter 0.1% gesunken war. Anschließend wurde das Produkt im Rotationsverdampfer vom Ethylacetat befreit. Das Produkt wurde als farbloser Feststoff erhalten.

**Beispiel 26: (6-Brom-1,1'-binaphthyl-2,2'-diyl)bis(oxycarbonyliminoethan-2,1-diyl) bis (2-methylacrylat)**

4.23 g 6-Brom-1,1'-binaphthyl-2,2'-diol (Herstellung siehe Beispiel 25) wurden mit 0.015 g Desmorapid Z und 0.01 g 2,6-Di-tert.-butyl-4-methylphenol in 25 mL Ethylacetat vorgelegt. Anschließend wurden 3.59 g KarenzMOI® zugetropft und die Mischung bei 80 °C gerührt, bis der Isocyanatgehalt unter 0.1 % gesunken war. Anschließend wurde

das Produkt im Rotationsverdampfer vom Ethylacetat befreit. Das Produkt wurde als farbloser Feststoff erhalten.

**Beispiel 27: (6-Cyan-1,1'-binaphthyl-2,2'-diyl)bis(oxycarbonyliminoethan-2,1-diyl) bisacrylat**

In einem Becherglas wurden 20.8 g 2-Naphtol, 22.2 g 6-Cyan-2-Naphthol und 1.5 g CuCl(OH)*TMEDA (hergestellt wie in Tetrahedron Letters 1994 (35), 7983-7984 beschrieben) intensiv vermischt und dann für 120 min auf 100 °C erhitzt. Das erhaltene Gemisch wurde mit 200 mL 10%-iger Ammoniak-Lösung und zweimal mit 200 mL Wasser gewaschen, getrocknet und chromatographisch gereinigt. Es wurden 4.80 g 6-Cyan-1,1'-binaphthyl-2,2'-diol erhalten.

4.10 g 6-Cyan-1,1'-binaphthyl-2,2'-diol wurden mit 0.015 g Desmorapid Z und 0.01 g 2,6-Di-tert.-butyl-4-methylphenol in 25 mL Ethylacetat vorgelegt. Anschließend wurden 3.72 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 28: (6-Cyan-1,1'-binaphthyl-2,2'-diyl)bis(oxycarbonyliminoethan-2,1-diyl) bis(2-methylacrylat)**

3.92 g 6-Cyan-1,1'-binaphthyl-2,2'-diol (Herstellung siehe Beispiel 27) wurden mit 0.015 g Desmorapid Z und 0.01 g 2,6-Di-tert.-butyl-4-methylphenol in 25 mL Ethylacetat vorgelegt. Anschließend wurden 3.90 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 29: (6-Brom-6'-cyan-1,1'-binaphthyl-2,2'-diyl)bis(oxycarbonyliminoethan-2,1-diyl) bisacrylat**

In einem Becherglas wurden 22.8 g 6-Brom-2-Naphtol, 15.8 g 6-Cyan-2-Naphthol und 1.4 g CuCl(OH)*TMEDA (hergestellt wie in Tetrahedron Letters 1994 (35), 7983-7984 beschrieben) intensiv vermischt und dann für 120 min auf 100 °C erhitzt. Das erhaltene Gemisch wurde mit 200 mL 10%-iger Ammoniak-Lösung und zweimal mit 200 mL Wasser gewaschen, getrocknet und chromatographisch gereinigt. Es wurden 4.70 g 6'-Brom-2,2'-dihydroxy-1,1'-binaphthyl-6-carbonitril erhalten.

4.54 g 6'-Brom-2,2'-dihydroxy-1,1'-binaphthyl-6-carbonitril wurden mit 0.015 g Desmorapid Z und 0.01 g 2,6-Di-tert.-butyl-4-methylphenol in 25 mL Ethylacetat vorgelegt. Anschließend wurden 3.28 g KarenzAOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Beispiel 30: (6-Brom-6'-cyan-1,1'-binaphthyl-2,2'-diyl)bis(oxycarbonyliminoethan-2,1-diyl)bis(2-methylacrylat)**

4.36 g 6'-Brom-2,2'-dihydioxy-1,1'-binaphthyl-6-carbonitril (Herstellung siehe Beispiel 29) wurden mit 0.015 g Desmorapid Z und 0.01 g 2,6-Di-tert.-butyl-4-methylphenol in 25 mL Ethylacetat vorgelegt. Anschließend wurden 3.46 g KarenzMOI® zugetropft und die Mischung bei 80 °C gerührt, bis der Isocyanatgehalt unter 0.1 % gesunken war. Anschließend wurde das Produkt im Rotationsverdampfer vom Ethylacetat befreit. Das Produkt wurde als farbloser Feststoff erhalten.

**Herstellung der weiteren Komponenten für die Photopolymer-Formulierung:**

**Herstellung von Polyol 1:**

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Herstellung des Urethanacrylats (Schreibmonomer) 1:2-({[3-(Methylsulfanyl)-phenyl]carbamoyl}oxy)ethylprop-2-enoat**

In einem 100 mL Rundkolben wurden 0.02 g 2,6-Di-tert.-butyl-4-methylphenol, 0.01 g Desmorapid Z, 11.7 g 3-(Methylthio)phenylisocyanat [28479-1-8] vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als farblose Flüssigkeit erhalten.

**Herstellung des Additivs 1 Bis(2,2,3,3,4,4,5,5,6,6,7,7-dodecafluorheptyl)-(2,2,4-trimethylhexan-1,6-diyl)biscarbamat**

In einem 50 mL Rundkolben wurden 0.02 g Desmorapid Z und 3.6 g Vestanat TMDI vorgelegt und auf 60 °C erwärmt. Anschließend wurden 11.9 g 2,2,3,3,4,4,5,5,6,6,7,7-Dodecafluorheptan-1-ol zugetropft und die Mischung weiter auf 60 °C gehalten, bis der Isocyanatgehalt unter 0.1 % gesunken war. Danach wurde abgekühlt. Das Produkt wurde als farbloses Öl erhalten.

**Herstellung des Vergleichsbeispiels 1 (Schreibmonomer): Phosphorothioyltris(oxybenzol-4,1-diylcarbamoyloxyethan-2,1-diyl)trisacrylat**

In einem 500 mL Rundkolben wurden 0.1 g 2,6-Di-tert.-butyl-4-methylphenol, 0.05 g Dibutylzinndilaurat sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und das Ethylacetat im Vakuum vollständig entfernt. Das Produkt wurde als teilkristalliner Feststoff erhalten.

**Herstellung des Vergleichsbeispiels 2 (Schreibmonomer): (Mischung aus (4-Methylbenzol-1,3-diyl)bis[carbamoyloxy-3-(biphenyl-2-yloxy)propan-2,1-diyl]bisacrylat und (4-Methylbenzol-1,3-diyl)bis[carbamoyloxy3-(biphenyl-2-yloxy)propan-1,2-diyl]bisacrylat und analoger Isomere):**

430.2 g Denacol EX 142 (Nagase-Chemtex, Japan), 129.7 g Acrylsäure, 1.18 g Triphenylphosphin und 0.006 g 2,6-Di-t.-butyl-4-methylphenol wurden in einem Dreihalskolben mit Rückflusskühler und Rührwerk vorgelegt. Zudem wurde Luft langsam durchgeleitet und auf 60 °C temperiert. Anschließend wird für 24 Stunden bei 90 °C gerührt. Man erhielt eine klare Flüssigkeit mit OH-Zahl = 157.8 mg KOH/g. 21.3 g dieses Zwischenprodukts und 5.2 g eines Gemischs aus 2,4- und 2,6- Tolylidendiisocyanat (Desmodur T80, Bayer MaterialScience AG, Leverkusen, Germany) wurden in einem Dreihalskolben mit Rückflusskühler und Rührwerk vorgelegt. Zudem wurde Luft langsam durchgeleitet und auf 60 °C temperiert. Nach anfänglicher Exothermie wurde das Produkt für 24 Stunden bei 60 °C gerührt. Es wurde ein klares, farbloses, gläsernes Produkt mit NCO = 0% erhalten.

**Herstellung der Medien zur Bestimmung der holographischen Eigenschaften Beispiel-Medium I**

3.38 g der Polyol-Komponente 1 wurden mit 2.00 g Beispiel 1 gemäß Formel (I), 2.00 g Urethanacrylat 2, 1.50 g Additiv 1, 0.10 g CGI 909, 0.026 g Farbstoff 1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 zugegeben und erneut gemischt. Schließlich wurden 0.01 g Fomrez UL 28 zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

Die Beispiel-Medien II - IX wurde hergestellt wie unter Beispiel-Medium I beschrieben. Es wurde - wie in Tabelle 1 aufgelistet - Beispiel 1 gegen das in der jeweiligen Zeile aufgeführte Beispiel in gleichem Gewichts-Anteil ersetzt.

**Vergleichs-Medium V-I**

3.38 g der Polyol-Komponente 1 wurden mit 2.00 g Vergleichsbeispiel 1, 2.00 g Urethanacrylat 1, 1.50 g Additiv 1, 0.10 g CGI 909, 0.010 g Farbstoff 1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 zugegeben und erneut gemischt. Schließlich wurden 0.01 g Fomrez UL 28 zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

**Vergleichs-Medium V-II**

3.38 g der Polyol-Komponente 1 wurden mit 2.00 g Vergleichsbeispiel 2, 2.00 g Urethanacrylat 1, 1.50 g Additiv 1, 0.10 g CGI 909, 0.010 g Farbstoff 1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 zugegeben und erneut gemischt. Schließlich wurden 0.01 g Fomrez UL 28 zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12

Stunden bei Raumtemperatur liegen gelassen und gehärtet.

**Herstellung von holographischen Medien auf einer Folienbeschichtungsanlage**

Die Bestimmung der Brechungsindexmodulation Δn in einem holographisch belichteten Photopolymer nach dem oben beschriebenen Verfahren gelingt am genauesten, wenn die Beugungseffizienz nicht völlig gesättigt sondern nahe bei 100% ist. Die Beugungseffizienz DE hängt dabei vom Produkt aus Δn und Schichtdicke d des Photopolymers ab. Bei den hier erhaltenen sehr hellen Hologrammen, die einen sehr hohen Brechungsindexkontrast Δn aufweisen, ist daher die Herstellung von Probekörpern mit sehr dünner Schichtdicke d erforderlich. Dazu wurde ein ausgewähltes Beispiel (Beispiel 3) in einer kontinuierlichen Beschichtungsanlage zu einem Photopolymer verarbeitet.

Figur 3 zeigt den schematischen Aufbau der verwendeten Beschichtungsanlage mit den folgenden Bauteilen:

| | |
|---|---|
| 1a, b | Vorratsbehälter |
| 2a, b | Dosiereinrichtung |
| 3a, b | Vakuumentgasungseinrichtung |
| 4a, b | Filter |
| 5 | Statischer Mischer |
| 6 | Beschichtungseinrichtung |
| 7 | Umlufttrockner |
| 8 | Trägersubstrat |
| 9 | Abdeckschicht |

Zur Herstellung der Photopolymer-Formulierung wurden in einem Rührbehälter 45.7 g Polyol 1 schrittweise mit 290.0 g Ethylacetat, 20.0 g Beispiel 1, 60.0 g Urethan-acrylat 1, 60.0 g Additiv 1, 0.10 g Fomrez UL 28, 1.80 g BYK® 310 und 0.52 g Farbstoff 1 versetzt und gemischt, so dass eine klare Lösung erhalten wurde. Dieses Gemisch wurde in den Vorratsbehälter 1a der Beschichtungsanlage eingebracht. In den zweiten Vorratsbehälter 1b wurde eine separat hergestellte klare Mischung aus 3.0 g CGI 909, 8.87 g Desmodur N 3900 und 2.22 g Butylacetat eingefüllt. Beide Komponenten wurden dann jeweils durch die Dosiereinrichtungen 2a und 2b im Verhältnis von 16.3 zu 1 (Vorratsbehälter la zu 1b) zu den Vakuumentgasungseinrichtungen 3a und 3b gefördert und entgast. Von hier aus wurden sie dann jeweils durch die Filter 4a und 4b in den statischen Mischer 5 geleitet, in dem die Vermischung der Komponenten zur Photopolymer-Formulierung erfolgte. Die erhaltene, flüssige Masse wurde dann im Dunklen der Beschichtungseinrichtung 6 zugeführt.

Bei der Beschichtungseinrichtung 6 handelte es sich im vorliegenden Fall um eine dem Fachmann bekannte Schlitzdüse. Alternativ kann aber auch ein Rakelsystem (Doctor Blade) oder ein Rollenantragssystem zum Einsatz kommen. Mit Hilfe der Beschichtungseinrichtung 6 wurde die Photopolymer-Formulierung bei einer Verarbeitungstemperatur von 20 °C auf ein Trägersubstrat 8 in der Form einer 36 μm dicke Polyethylenterephthalatfolie appliziert und für 5.8 Minuten bei einer Vernetzungstemperatur von 80°C in einem Umlufttrockner 7 getrocknet. Dabei wurde ein Medium in der Form eines Films erhalten, der dann mit einer 40 μm Polyethylenfolie als Abdeckschicht 9 versehen und aufgewickelt wurde.

Die erzielte Schichtdicke des Films beträgt 6 - 8 μm.

**Holographische Prüfung:**

Die wie beschrieben hergestellten Medien wurden mittels einer Messordnung gemäß Figur 1 in der oben beschriebenen Weise auf ihre holographischen Eigenschaften geprüft. Dabei ergaben sich folgende Messwerte für An bei einer fixen Dosis 36 mJ/cm$^2$:

Tab. 1: Holographische Bewertung ausgewählter Beispiele

| Beispiel-Medium | Beispiel gemäß Formel (I) | Δn |
|---|---|---|
| I | 1 | 0.043 |
| II | 3 | 0.050 |
| III | 4 | 0.051 |
| IV | 6 | 0.050 |
| V | 7 | 0.040 |

(fortgesetzt)

| Beispiel-Medium | Beispiel gemäß Formel (I) | $\Delta n$ |
|---|---|---|
| VI | 8 | 0.040 |
| VII | 19 | 0.038 |
| VIII | 20 | 0.037 |
| IX | 22 | 0.038 |
| X | 3 | 0.060 |

Tab. 2: Holographische Bewertung ausgewählter Vergleichs-Medien

| Vergleichs-Medium | $\Delta n$ |
|---|---|
| V-I | 0.035 |
| V-II | 0.035 |

Die gefundenen Werte für die Beispiel-Medien I bis IX zeigen, dass die in den Photopolymer-Formulierungen eingesetzten erfindungsgemäßen Verbindungen der Formel (I) für die Verwendung in holographischen Medien mit sehr hoher Brechungsindexmodulation An sehr gut geeignet sind. Die Vergleichs-Medien V-1 und V-2 weisen keine erfindungsgemäßen Verbindung der Formel (I) auf und weisen in holographischen Medien geringere Werte für $\Delta n$ auf. Mit dem Beispiel-Medium X kann gezeigt werden, dass die eingesetzten erfindungsgemäßen Verbindungen der Formel (I) eine sehr hohe Brechungsindexmodulation $\Delta n$ aufweisen.

## Patentansprüche

**1.** Verbindung gemäß Formel (I)

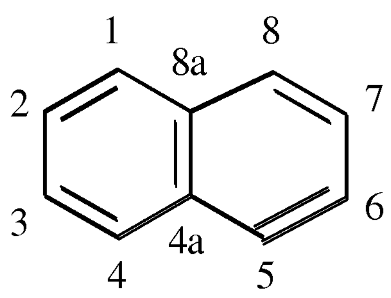

Formel (I)

a) die an wenigstens einem der Kohlenstoffatome 1, 2, 3, 4, 5, 6, 7, 8 mit einem Rest $R_{Arcyl}$ der Formel (II) substituiert ist,

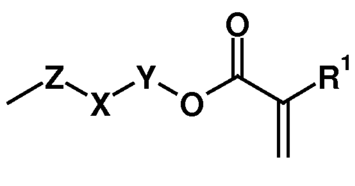

Formel (II)

wobei in der Formel (II)

$R^1$ Wasserstoff oder eine ($C_1$-$C_6$)-Alkylgruppe,
X eine Carbonamid (-C(O)N-) oder eine Carbonester (-C(O)O-) oder eine Sulfonamid (-$SO_2$N-) Gruppe,
Y ein gesättigter oder ungesättigter oder linearer oder verzweigter gegebenenfalls substituierter 2-10 C-Atome aufweisender Rest oder ein bis zu fünf (-$CH_2$-$CH_2$-O-)- oder (-C($CH_3$)H-$CH_2$-O-)-Gruppen aufweisender Polyether oder ein bis zu fünf Stickstoff-Atome aufweisendes Polyamin, und
Z Sauerstoff oder Schwefel ist,

b) und die Verbindung der Formel (I) an wenigstens einem weiteren Kohlenstoffatom 1, 2, 3, 4, 5, 6, 7, 8 mit einem Rest der Formel (III) substituiert ist

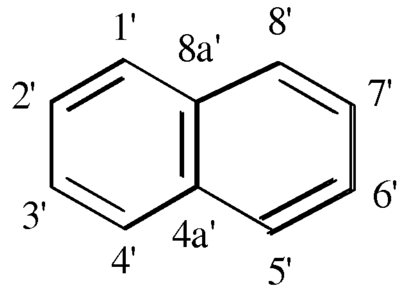

Formel (III)

wobei in der Formel (III)
die Kohlenstoffatome der Verbindung der Formel (III) jeweils unabhängig mit Wasserstoff, Halogen, einer Cyano-Gruppe, einer Nitro-Gruppe oder einer gegebenenfalls substituierten Alkyl-, Alkenyl-, Alkinyl-, Aralkyl-, Aryl- oder Heteroaryl-Gruppe oder einer gegebenenfalls substituierten Alkylthio-Gruppe oder einer beliebig substituierten Carbamoyl-Gruppe, die auch verbrückend an einen Rest der Formel (I) gebunden sein kann, oder einer Trifluormethyl-Gruppe oder einer Trifluormethoxy-Gruppe oder einem Rest $R_{Arcyl'}$ der Formel (IV),

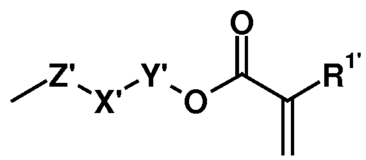

Formel (IV)

wobei in der Formel (IV)

$R^{1'}$ Wasserstoff oder eine (C1-C6)-Alkylgruppe,
X' eine Carbonamid (-C(O)N-) oder eine Carbonester (-C(O)O-) oder eine Sulfonamid (-$SO_2$N-) Gruppe,
Y' ein gesättigter oder ungesättigter oder linearer oder verzweigter gegebenenfalls substituierter 2-10 C-Atome aufweisender Rest oder ein bis zu fünf (-$CH_2$-$CH_2$-O-)- oder (-C($CH_3$)H-$CH_2$-O-)-Gruppen aufweisender Polyether oder ein bis zu fünf Stickstoff-Atome aufweisendes Polyamin, und
Z' Sauerstoff oder Schwefel ist,

substituiert sind und
c) die übrigen Kohlenstoffatome der Verbindung der Formel (I) jeweils unabhängig mit Wasserstoff, Halogen, einer Cyano-Gruppe, einer Nitro-Gruppe oder einer gegebenenfalls substituierten Alkyl-, Alkenyl-, Alkinyl-, Aralkyl-, Aryl- oder Heteroaryl-Gruppe oder einer gegebenenfalls substituierten Alkylthio-Gruppe oder einer Trifluormethyl-Gruppe oder einer Trifluormethoxy-Gruppe substituiert sind.

**2.** Verbindung nach Anspruch 1, **dadurch gekennzeichnet, dass** sie an dem Kohlenstoffatom der Position 5 in der Formel (I) mit dem Rest der Formel (III) substituiert ist, wobei der Rest der Formel (III) bevorzugt über das Kohlenstoffatom der Position 8’ an das Kohlenstoffatom der Position 5 gebunden ist.

**3.** Verbindung nach einem der Ansprüche 1 oder 2, **dadurch gekennzeichnet, dass** sie an dem Kohlenstoffatom der Position 6 in der Formel (I) mit dem Rest R der Formel (II) substituiert ist.

**4.** Verbindung nach einem der Ansprüche 1 bis 3, **dadurch gekennzeichnet, dass** der Rest der Formel (III) an dem Kohlenstoffatom der Position 7’ mit dem Rest $R_{Acryl'}$ der Formel (IV) substituiert ist.

**5.** Verbindung nach einem der Ansprüche 1 bis 4, **dadurch gekennzeichnet, dass** in dem Rest $R_{Arcyl}$ X für Carbonamid steht und / oder in dem Rest $R_{ArCyl'}$ X’ für Carbonamid steht.

**6.** Verbindung nach einem der Ansprüche 1 bis 5, **dadurch gekennzeichnet, dass** in dem Rest $R_{Acryl}$ $R_1$ Wasserstoff oder ein $CH_3$-Rest und / oder in dem Rest $R_{Acryl'}$ $R_{1'}$ Wasserstoff oder ein $CH_3$-Rest ist.

**7.** Verbindung nach einem der Ansprüche 1 bis 6, **dadurch gekennzeichnet, dass** in dem Rest $R_{Acryl}$ Y für eine $-CH_2-CH_2-$ Gruppe steht und / oder in dem der Rest $R_{Acryl'}$ Y’ für eine $-CH_2-CH_2-$Gruppe steht.

**8.** Verbindung nach einem der Ansprüche 1 bis 7, **dadurch gekennzeichnet, dass** in dem Rest $R_{Acryl}$ Z für Sauerstoff und / oder in dem Rest $R_{Acryl'}$ Z’ für Sauerstoff steht.

**9.** Photopolymer-Formulierung umfassend Matrixpolymere, Schreibmonomere und Photoinitiatoren, wobei die Schreibmonomere eine Verbindung gemäß einem der Ansprüche 1 bis 8 umfassen.

**10.** Holografisches Medium umfassend Matrixpolymere, Schreibmonomere und Photoinitiatoren, wobei die Schreibmonomere eine Verbindung gemäß einem der Ansprüche 1 bis 8 umfassen.

**11.** Holografisches Medium nach Anspruch 10, **dadurch gekennzeichnet, dass** die Matrixpolymere vernetzt, bevorzugt dreidimensional vernetzt und ganz besonders bevorzugt dreidimensional vernetzte Polyurethane sind.

**12.** Holografisches Medium nach einem der Ansprüche 10 oder 11, **dadurch gekennzeichnet, dass** es wenigstens ein Fluorurethan als Additiv umfasst.

**13.** Holografisches Medium nach einem der Ansprüche 10 bis 12, **dadurch gekennzeichnet, dass** es ein Films ist, der bevorzugt eine Filmdicke 0.5 $\mu$m bis 200 $\mu$m, weiter bevorzugt von 0.8 $\mu$m bis 50 $\mu$m und besonders bevorzugt von 1 $\mu$m bis 25 $\mu$m aufweisen kann.

**14.** Holografisches Medium nach einem der Ansprüche 10 bis 13, **dadurch gekennzeichnet, dass** wenigstens ein Hologramm einbelichtet ist.

**15.** Display umfassend ein holografische Medium nach einem der Ansprüche 10 bis 14.

## Claims

**1.** Compound of formula (I)

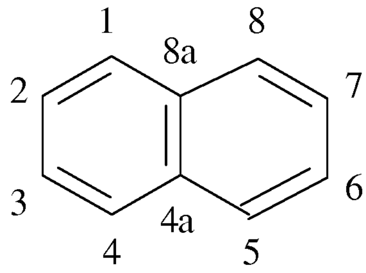

Formula (I):

a) which is substituted at at least one of the carbon atoms 1, 2, 3, 4, 5, 6, 7, 8 with a moiety $R_{acryl}$ of formula (II)

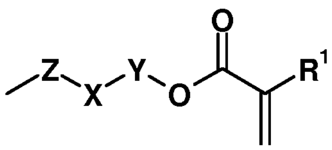

Formula (II):

where in said formula (II)

$R^1$ is hydrogen or a ($C_1$-$C_6$)-alkyl group,
X is a carboxamide (-C(O)N-) or a carboxylic ester (-C(O)O-) or a sulphonamide (-$SO_2$N-) group,
Y is a saturated or unsaturated or linear or branched optionally substituted moiety having 2-10 carbon atoms or a polyether having from one up to five (-$CH_2$-$CH_2$-O-)- or (-C($CH_3$)H-$CH_2$-O-)- groups or a polyamine having from one to five nitrogen atoms, and
Z is oxygen or sulphur,

b) and the compound of formula (I) is at not less than one further carbon atom, 1, 2, 3, 4, 5, 6, 7, 8 substituted with a moiety of formula (III)

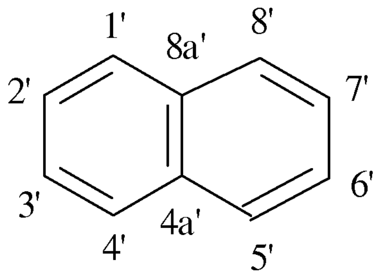

Formula (III)

where in said formula (III)
the carbon atoms of the compound of formula (III) are each independently substituted with hydrogen, halogen, a cyano group, a nitro group or an optionally substituted alkyl, alkenyl, alkynl, aralkyl, aryl or heteroaryl group or an optionally substituted alkylthio group or any substituted carbamoyl group, which also may be linked bridgingly to a moiety of formula (I), or a trifluoromethyl group or a trifluoromethoxy group or a moiety $R_{acryl}$' of formula (IV),

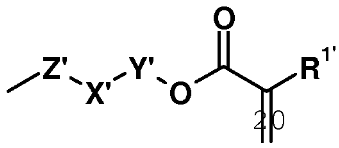

Formula (IV):

where in said formula (IV)

$R^{1'}$ is hydrogen or a (C1-C6)-alkyl group,
X' is a carboxamide (-C(O)N-) or a carboxylic ester (-C(O)O-) or a sulphonamide ($-SO_2N-$) group,
Y' is a saturated or unsaturated or linear or branched optionally substituted moiety having 2-10 carbon atoms or a polyether having from one to five ($-CH_2-CH_2-O-$)- or ($-C(CH_3)H-CH_2-O-$)- groups or a polyamine having from one to five nitrogen atoms, and
Z is oxygen or sulphur,

c) the remaining carbon atoms of the compound of formula (I) are each independently substituted with hydrogen, halogen, a cyano group, a nitro group or an optionally substituted alkyl, alkenyl, alkynyl, aralkyl, aryl or heteroaryl group or an optionally substituted alkylthio group or a trifluoromethyl group or a trifluoromethoxy group.

**2.** Compound according to Claim 1, **characterized in that** it is substituted with the moiety of formula (III) on the carbon atom in position 5 of formula (I), wherein the moiety of formula (III) is preferably bonded to the carbon atom in position 5 via the carbon atom in position 8'.

**3.** Compound according to either of Claims 1 and 2, **characterized in that** it is substituted with the moiety R of formula (II) on the carbon atom in position 6 of formula (I).

**4.** Compound according to any of Claims 1 to 3, **characterized in that** the moiety of formula (III) is substituted with the moiety $R_{acryl'}$ of formula (IV) on the carbon atom in position 7'.

**5.** Compound according to any of Claims 1 to 4, **characterized in that** X is carboxamide in moiety $R_{acryl}$ and/or X' is carboxamide in moiety $R_{acryl'}$.

**6.** Compound according to any of Claims 1 to 5, **characterized in that** $R_1$ is hydrogen or a $CH_3$ moiety in moiety $R_{acryl}$ and/or $R_{1'}$ is hydrogen or a $CH_3$ moiety in moiety $R_{acryl'}$.

**7.** Compound according to any of Claims 1 to 6, **characterized in that** Y is a $-CH_2-CH_2$ group in moiety $R_{acryl}$ and/or $Y^1$ is a $-CH_2-CH_2$ group in moiety $R_{acryl'}$.

**8.** Compound according to any of Claims 1 to 7, **characterized in that** Z is oxygen in moiety $R_{acryl}$ and/or Z' is oxygen in moiety $R_{acryl'}$.

**9.** Photopolymer formulation comprising matrix polymers, writing monomers and photoinitiators, wherein the writing monomers comprise a compound according to any of Claims 1 to 8.

**10.** Holographic medium comprising matrix polymers, writing monomers and photoinitiators, wherein the writing monomers comprise a compound according to any of Claims 1 to 8.

**11.** Holographic medium according to Claim 10, **characterized in that** the matrix polymers are crosslinked matrix polymers, preferably three-dimensionally crosslinked matrix polymers and most preferably are three-dimensionally crosslinked polyurethanes.

**12.** Holographic medium according to either of Claims 10 and 11, **characterized in that** it comprises at least a fluorourethane as additive.

**13.** Holographic medium according to any of Claims 10 to 12, **characterized in that** it is a film, preferably with a film

thickness of 0.5 µm to 200 µm, more preferably with a film thickness of 0.8 µm to 50 µm and yet more preferably with a film thickness of 1 µm to 25 µm.

**14.** Holographic medium according to any of Claims 10 to 13, **characterized in that** it contains at least one exposed hologram.

**15.** Display comprising a holographic medium according to any of Claims 10 to 14.

**Revendications**

**1.** Composé selon la formule (I)

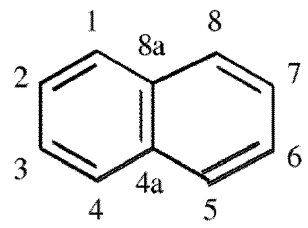

Formule (I)

a) qui est substitué sur au moins un des atomes de carbone 1, 2, 3, 4, 5, 6, 7, 8 avec un radical $R_{Arcyl}$ de formule (II),

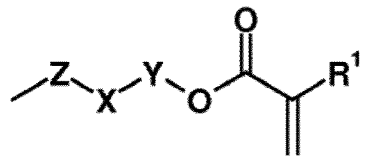

Formule (II)

dans la formule (II),

$R^1$ représentant l'hydrogène ou un groupe alkyle en ($C_1$-$C_6$),
X représentant un groupe carbonamide (-C(O)N-) ou ester carbonique (-C(O)O-) ou sulfonamide ($-SO_2N-$) ,
Y représentant un radical saturé ou insaturé ou linéaire ou ramifié, éventuellement substitué, comprenant 2 à 10 atomes C, ou un polyéther comprenant jusqu'à cinq groupes ($-CH_2-CH_2-O-$) ou ($-C(CH_3)H-CH_2-O-$), ou une polyamine comprenant jusqu'à cinq atomes d'azote, et
Z représentant l'oxygène ou le soufre,

b) et le composé de formule (I) est substitué sur au moins un autre atome de carbone 1, 2, 3, 4, 5, 6, 7, 8 avec un radical de formule (III)

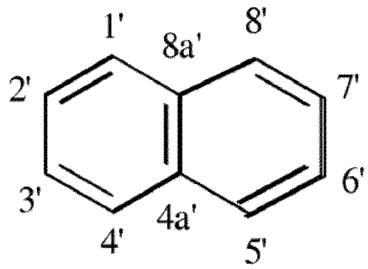

Formule (III)

dans la formule (III),

les atomes de carbone du composé de formule (III) pouvant chacun indépendamment être substitués avec un hydrogène, un halogène, un groupe cyano, un groupe nitro ou un groupe alkyle, alcényle, alcynyle, aralkyle, aryle ou hétéroaryle éventuellement substitué, ou un groupe alkylthio éventuellement substitué, ou un groupe carbamoyle substitué de manière quelconque, qui peut également être relié par un pont à un radical de formule (I), ou un groupe trifluorométhyle ou un groupe trifluorométhoxy ou un radical $R_{Arcyl'}$ de formule (IV)

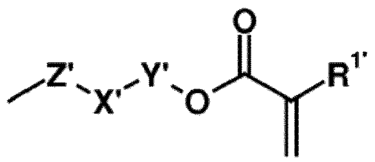

Formule (IV)

dans la formule (IV),

$R^{1'}$ représentant l'hydrogène ou un groupe alkyle en ($C_1$-$C_6$),
X' représentant un groupe carbonamide (-C(O)N-) ou ester carbonique (-C(O)O-) ou sulfonamide (-$SO_2$N-),
Y' représentant un radical saturé ou insaturé ou linéaire ou ramifié, éventuellement substitué, comprenant 2 à 10 atomes C, ou un polyéther comprenant jusqu'à cinq groupes (-$CH_2$-$CH_2$-O-) ou (-C($CH_3$)H-$CH_2$-O-) ou une polyamine comprenant jusqu'à cinq atomes d'azote, et
Z' représente l'oxygène ou le soufre,

et

c) les atomes de carbone restants du composé de formule (I) sont chacun indépendamment substitués avec un hydrogène, un halogène, un groupe cyano, un groupe nitro ou un groupe alkyle, alcényle, alcynyle, aralkyle, aryle ou hétéroaryle éventuellement substitué, ou un groupe alkylthio éventuellement substitué ou un groupe trifluorométhyle ou un groupe trifluorométhoxy.

**2.** Composé selon la revendication 1, **caractérisé en ce qu'**il est substitué sur l'atome de carbone de la position 5 dans la formule (I) avec le radical de formule (III), le radical de formule (III) étant de préférence relié par l'atome de carbone de la position 8' à l'atome de carbone de la position 5.

**3.** Composé selon l'une quelconque des revendications 1 ou 2, **caractérisé en ce qu'**il est substitué sur l'atome de carbone de la position 6 dans la formule (I) avec le radical R de formule (II).

**4.** Composé selon l'une quelconque des revendications 1 à 3, **caractérisé en ce que** le radical de formule (III) est substitué sur l'atome de carbone de la position 7' avec le radical $R_{Acryl'}$ de formule (IV) .

**5.** Composé selon l'une quelconque des revendications 1 à 4, **caractérisé en ce que**, dans le radical $R_{Acryl}$, X représente un carbonamide et/ou, dans le radical $R_{Acryl'}$, X' représente un carbonamide.

6. Composé selon l'une quelconque des revendications 1 à 5, **caractérisé en ce que**, dans le radical $R_{Acryl}$, $R_1$ représente l'hydrogène ou un radical $CH_3$ et/ou, dans le radical $R_{Acryl'}$, $R_{1'}$ représente l'hydrogène ou un radical $CH_3$.

7. Composé selon l'une quelconque des revendications 1 à 6, **caractérisé en ce que**, dans le radical $R_{Acryl}$, Y représente un groupe $-CH_2-CH_2-$ et/ou, dans le radical $R_{Acryl'}$, Y' représente un groupe $-CH_2-CH_2-$.

8. Composé selon l'une quelconque des revendications 1 à 7, **caractérisé en ce que**, dans le radical $R_{Acryl}$, Z représente l'oxygène et/ou, dans le radical $R_{Acryl'}$, Z' représente l'oxygène.

9. Formulation de photopolymère comprenant des polymères de matrice, des monomères d'écriture et des photoinitiateurs, dans laquelle les monomères d'écriture comprennent un composé selon l'une quelconque des revendications 1 à 8.

10. Support holographique comprenant des polymères de matrice, des monomères d'écriture et des photoinitiateurs, dans lequel les monomères d'écriture comprennent un composé selon l'une quelconque des revendications 1 à 8.

11. Support holographique selon la revendication 10, **caractérisé en ce que** les polymères de matrice sont réticulés, de préférence réticulés tridimensionnellement et de manière tout particulièrement des polyuréthanes réticulés tridimensionnellement.

12. Support holographique selon l'une quelconque des revendications 10 ou 11, **caractérisé en ce qu'**il comprend au moins un fluorouréthane en tant qu'additif.

13. Support holographique selon l'une quelconque des revendications 10 à 12, **caractérisé en ce qu'**il s'agit d'un film, qui peut de préférence présenter une épaisseur de film de 0,5 $\mu$m à 200 $\mu$m, de manière davantage préférée de 0,8 $\mu$m à 50 $\mu$m et de manière particulièrement préférée de 1 $\mu$m à 25 $\mu$m.

14. Support holographique selon l'une quelconque des revendications 10 à 13, **caractérisé en ce qu'**au moins un hologramme est exposé.

15. Écran comprenant un support holographique selon l'une quelconque des revendications 10 à 14.

Figur 1:

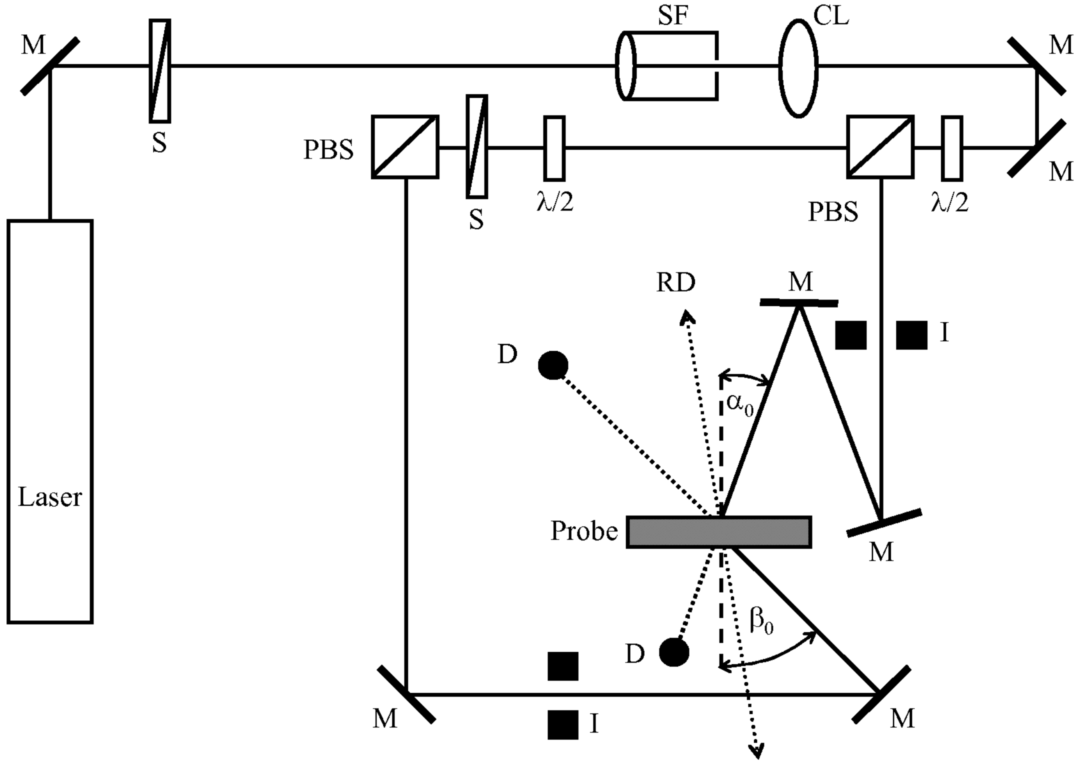

**Figur 2:**

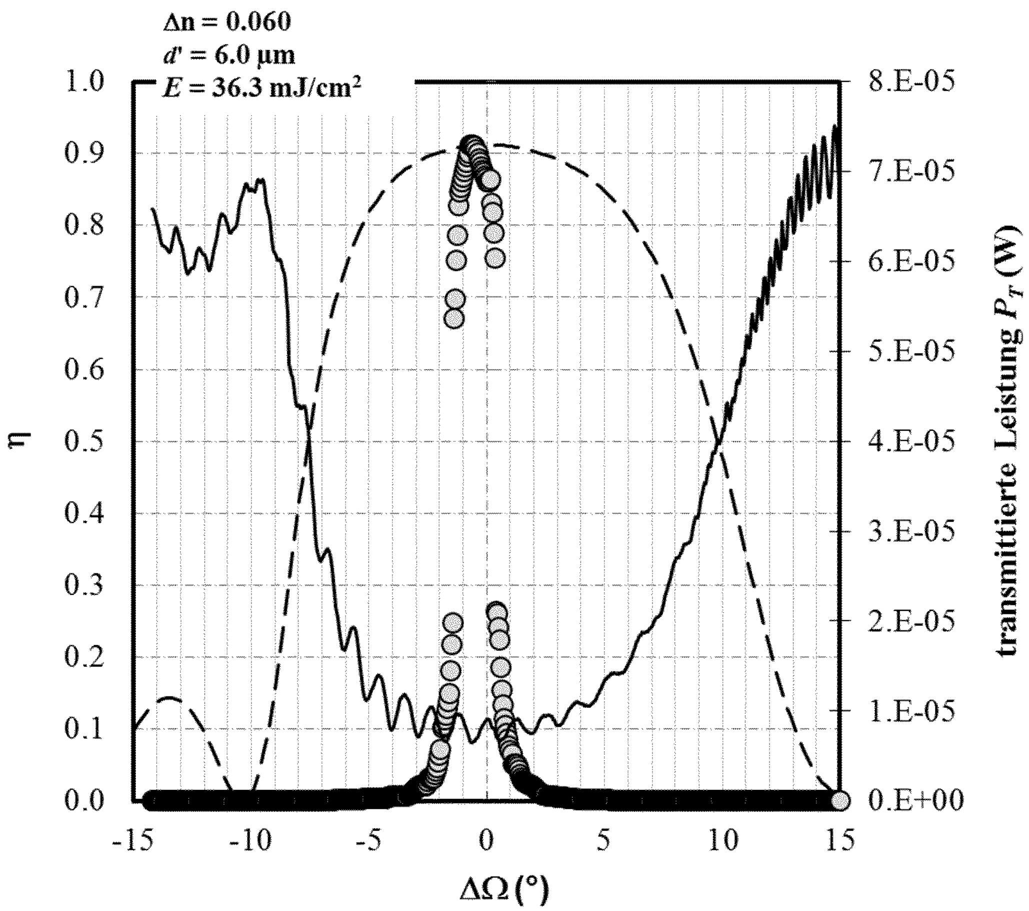
Δn = 0.060
d' = 6.0 µm
E = 36.3 mJ/cm²
η
transmittierte Leistung $P_T$ (W)
ΔΩ (°)
1.0
0.9
0.8
0.7
0.6
0.5
0.4
0.3
0.2
0.1
0.0
8.E-05
7.E-05
6.E-05
5.E-05
4.E-05
3.E-05
2.E-05
1.E-05
0.E+00
-15
-10
-5
0
5
10
15

**Figur 3:**

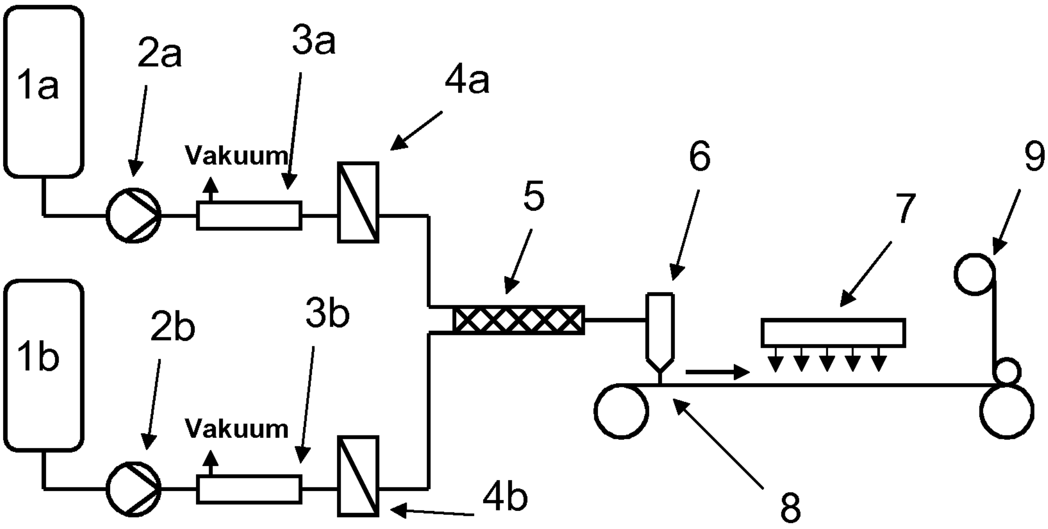


Figur 3: Schematischer Aufbau einer verwendeten kontinuierlichen Film-Beschichtungsanlage (als Rolle-zu-Rolle-Verfahren)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2154128 A
- WO P2013014533 A
- WO 2011054818 A
- EP 0223587 A
- WO 2012062655 A
- US 2009174919 A1
- WO 2012035058 A
- EP 14155599 A


### In der Beschreibung aufgeführte Nicht-Patentliteratur


- **P. HARIHARAN.** Optical Holography. Cambridge University Press, 1996
- Azine Dyes. **H. BERNETH.** Ullmann's Encyclopedia of Industrial Chemistry. Wiley-VCH Verlag, 2008
- Methine Dyes and Pigments. **H. BERNETH.** Ullmann's Encyclopedia of Industrial Chemistry. Wiley-VCH Verlag, 2008
- Triarylmethane and Diarylmethane Dyes. **T. GESSNER ; U. MAYER.** Ullmann's Encyclopedia of Industrial Chemistry. Wiley-VCH Verlag, 2000
- Virtual Computational Chemistry Laboratory. *J. Comput. Aid. Mol. Des.,* 2005, vol. 19, 453, http://www.vcclab.org berechnet
- **CUNNINGHAM et al.** *RadTech'98 North America UV/EB Conference Proceedings,* 19. April 1998
- Chemistry & Technology of UV & EB Formulations For Coatings. Inks & Paints. SITA Technology, 1991, vol. 3, 61-328
- **H. KOGELNIK.** *The Bell System Technical Journal,* November 1969, vol. 48 (9), 2909, , 2947
- *Tetrahedron Letters,* 1994, vol. 35 (43), 7983-4
- *Tetrahedron Letters,* 1994, vol. 35, 7983-7984